(19) Europäisches Patentamt
European Patent Office
Office européen des brevets

(11) Publication number: **0 167 011**
**A2**

(12) # EUROPEAN PATENT APPLICATION

(21) Application number: 85107011.0

(22) Date of filing: 05.06.85

(51) Int. Cl.⁴: C 07 D 499/68
C 07 D 501/20, C 07 D 498/04
C 07 D 471/04, C 07 C 103/44
C 07 C 125/00, C 07 C 127/15
//(C07D498/04, 265:00, 205:00),
(C07D471/04, 221:00, 205:00)

(30) Priority: 12.06.84 GB 8414986
08.01.85 GB 8500464

(43) Date of publication of application:
08.01.86 Bulletin 86/2

(84) Designated Contracting States:
BE CH DE FR GB IT LI NL SE

(71) Applicant: BEECHAM GROUP PLC
Beecham House Great West Road
Brentford Middlesex TW8 9BD(GB)

(72) Inventor: Smale, Terence Charles
28 St. Leonards Road
Epsom Downs Surrey(GB)

(72) Inventor: Milner, Peter Henry
12 The Marts
Rudgwick West Sussex RH12 3HH(GB)

(72) Inventor: Stachulski, Andrew Valentine
3 Ridgeway Court Ridgeway Road
Redhill Surrey, RH1 6PG(GB)

(74) Representative: Lockwood, Barbara Ann et al,
Beecham Pharmaceuticals Biosciences Research Centre
Great Burgh Yew Tree Bottom Road
Epsom Surrey KT18 5XQ(GB)

(54) **Beta-lactam compounds, intermediates thereof, their use in preparing beta-lactams having a diketopiperazine group.**

(57) Process for preparation of 6/7α substituted β-lactams having a diketopiperazine group in the side-chain, which comprises ring-closure of a compound of partial structure:

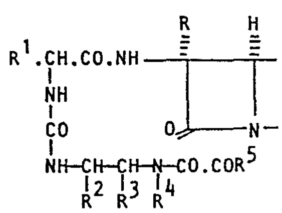

R = alkylthio, arythio, methoxy, hydroxymethyl amino or formamido.

EP 0 167 011 A2

B1658

TITLE MODIFIED
see front page

CHEMICAL PROCESS

This invention relates to a chemical process for preparing a class of β-lactam compounds. The β-lactam compounds produced by this process have antibacterial activity and are therefore useful in the treatment of bacterial infections in animals, especially mammals including man caused by a wide range of organisms, particularly Gram-negative organisms.

The present invention provides a process for the preparation of a β-lactam compound having the partial structure (I):

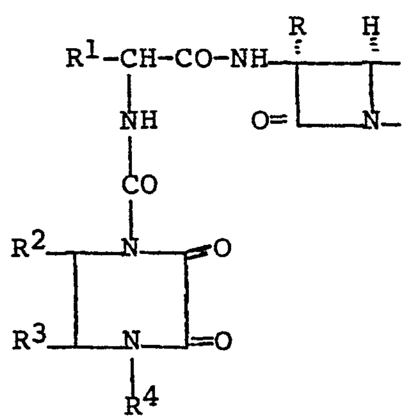

(I)

wherein: R represents $C_{1-6}$ alkylthio, arylthio, methoxy, hydroxymethyl, amino or formamido;

$R^1$ represents hydrocarbon or heterocyclyl;

$R^2$ and $R^3$ are the same or different and each represents hydrogen, optionally substituted $C_{1-6}$ alkyl, halogen, aryl amino, hydroxy or $C_{1-6}$ alkoxy or $R^2$ and $R^3$ form the residue of a 5- or 6-membered carbocyclic or heterocyclic ring; and

$R^4$ represents hydrogen, or hydrocarbon;

which process comprises cyclising a compound of formula (II):

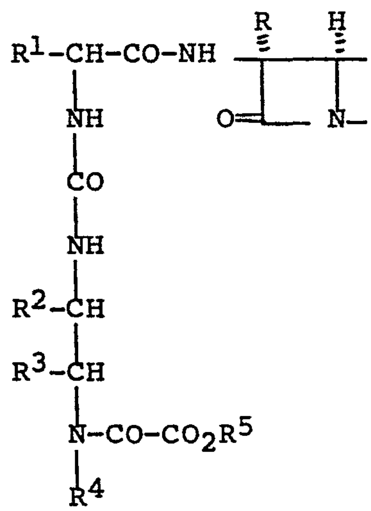

(II)

wherein R, $R^1$, $R^2$, $R^3$ and $R^4$ are as defined with respect to formula (I), and $R^5$ represents hydrogen or a group which allows the $-CO_2R^5$ group to effect N-acylation.

In particular, the compounds of formula (I) produced by the process of this invention may have the formula (III):

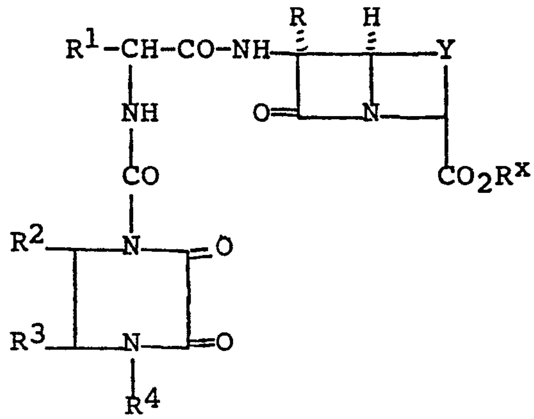

(III)

wherein R, $R^1$, $R^2$, $R^3$ and $R^4$ are as defined with respect to formula (I);

$R^x$ is hydrogen or a carboxyl blocking group;

and Y is:

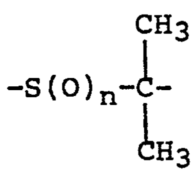 , $-S(O)_n-CH_2-$ ,

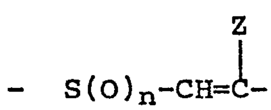 , 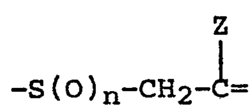 ,

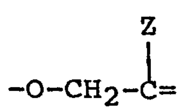 , 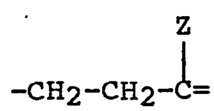 ,

wherein n is zero, 1 or 2 and Z represents hydrogen, halogen, or an organic group such as $C_{1-4}$ alkoxy, $-CH_2Q$ or -CH=CH-Q wherein Q represents hydrogen, halogen, hydroxy, mercapto, cyano, carboxy, carboxylic ester, carbamoyloxy, $C_{1-4}$ alkyloxy, acyloxy, aryl, a

heterocyclyl group bonded *via* carbon, a heterocyclylthio group or a nitrogen containing heterocyclic group bonded *via* nitrogen.

Preferred values for Y in the compounds of formula (III) are $-S-C(CH_3)_2-$ and $-S-CH_2CZ=$, *ie* when the compound of formula (III) is a derivative of a penicillin or cephalosporin.

Those compounds of the formula (III) wherein $R^x$ is a readily removable carboxyl protecting group or a non-pharmaceutically acceptable salt are primarily useful as intermediates in the preparation of compounds wherein $R^x$ is hydrogen or a pharmaceutically acceptable salting ion, which compounds are useful as antibacterial agents. Also included within the readily removable carboxyl protecting groups $R^x$ are pharmaceutically acceptable in vivo hydrolysable ester groups.

Those compounds of the formula (III) wherein Y is -SO-CH=CZ- are primarily useful as intermediates in the preparation of compounds wherein Y is $-SO-CH_2-CZ=$.

The compounds of formula (II) and (III) wherein R is $C_{1-6}$ alkylthio, arylthio or amino are useful intermediates for conversion into compounds wherein R is methoxy or formamido.

Although the purity of the intermediate compounds of the present invention is less critical than for compounds used in pharmaceutical compositions it will readily be understood that the substantially pure form is preferred as for the β-lactam antibiotic compounds. Preferably, whenever possible, the compounds of the present invention are obtained in crystalline form.

The term 'hydrocarbon' includes groups having up to 18 carbon atoms, suitably up to 10 carbon atoms, conveniently up to 6 carbon atoms. Suitable hydrocarbon groups include $C_{1-6}$ alkyl, $C_{2-6}$ alkenyl, $C_{2-6}$ alkynyl, $C_{3-7}$ cycloalkyl, $C_{3-7}$ cycloalkyl $(C_{1-6})$-alkyl, aryl, and aryl$(C_{1-6})$alkyl.

The alkyl group may be straight or branched chain and may contain, for example, up to 12 carbon atoms, suitably from 1 to 6 carbon atoms. In particular the group may be substituted methyl, ethyl, n-, or iso-propyl, or n-, sec-, iso- or tert-butyl.

The term 'heterocyclyl' includes single or fused rings comprising up to four hetero atoms in the ring selected from oxygen, nitrogen and sulphur and optionally substituted with up to three halogen, $C_{1-6}$ alkyl, $C_{1-6}$ alkoxy, halo-$(C_{1-6})$-alkyl, hydroxy, amino, carboxy, $C_{1-6}$ alkoxycarbonyl, $C_{1-6}$ alkoxycarbonyl$(C_{1-6})$ alkyl, aryl or oxo groups.

Suitably the heterocyclic ring comprises from 4 to 7 ring atoms, preferably 5 to 6 atoms.

When used herein the term 'aryl' includes phenyl and naphthyl optionally substituted with up to five, preferably up to three, groups selected from halogen, $C_{1-6}$ alkyl, phenyl, $C_{1-6}$ alkoxy, halo$(C_{1-6})$ alkyl, hydroxy, amino, nitro, carboxy, $C_{1-6}$ alkoxycarbonyl, $C_{1-6}$ alkoxycarbonyl-$(C_{1-6})$-alkyl, $C_{1-6}$ alkylcarbonyloxy, $C_{1-6}$ alkylcarbonyl or $C_{1-6}$ alkoxycarbonyloxy groups.

Suitable optional substituents for the hydrocarbon, heterocyclic groups and organic radicals include $C_{1-6}$ alkyl, heterocyclyl, amino, $C_{1-6}$ alkanoyl-amino, mono, and

di-($C_{1-6}$) alkylamino, hydroxy, $C_{1-6}$ alkoxy, mercapto, $C_{1-6}$ alkylthio, heterocyclyl-thio, arylthio, sulphamoyl, carbamoyl, amidino, guanidino, nitro, chloro, bromo, fluoro, carboxy and salts and esters thereof, $C_{1-6}$ alkanoyloxy, aryl-carbonyl and heterocyclylcarbonyl.

Suitable carboxyl-blocking derivatives for the group $-CO_2R^x$ in formula (III) include salts and ester derivatives of the carboxylic acid. The derivative is preferably one which may readily be cleaved at a later stage of the reaction. Suitable salts include metal salts, such as those with sodium, potassium and lithium, and tertiary amine salts, such as those with trilower-alkylamines, N-ethylpiperidine, 2,6-lutidine, pyridine, N-methylpyrrolidine, dimethylpiperazine. A preferred salt is with triethylamine.

Suitable ester-forming carboxyl-protecting groups are those which may be removed under conventional conditions. Such groups for $R^x$ include benzyl, p-methoxybenzyl, 2,4,6-trimethylbenzyl, 3,5-di-t-butyl-4-hydroxy-benzyl, benzoylmethyl, p-nitrobenzyl, 4-pyridylmethyl, 2,2,2-trichloroethyl, 2,2,2-tribromoethyl, allyl, acetonyl, t-butyl, t-amyl, diphenylmethyl, triphenylmethyl, adamantyl, 2-benzyl-oxyphenyl, 4-methylthiophenyl, tetrahydrofur-2-yl, tetrahydropyran-2-yl, pentachlorophenyl, p-toluene-sulphonylethyl, methoxymethyl, a silyl, stannyl or phosphorus-containing group, an oxime radical of formula $-N{=}CHR^o$
wherein
$R^o$ is aryl or heterocyclic,
or an _in vivo_ hydrolysable ester radical.

The carboxyl group may be regenerated from any of the above esters by usual methods appropriate to the particular $R^x$ group, for example, acid - and base - catalysed hydrolysis, or by enzymically - catalysed hydrolysis, or by hydrogenation.

Suitable pharmaceutically acceptable salts of the compounds of formula (III) include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine or quinoline.

Suitable values for Q in the compounds of the formula (III) include the acetoxy, heterocyclylthio group, and nitrogen containing heterocyclic group bonded <u>via</u> nitrogen.

More suitably Q represents the acetoxy or heterocyclylthio group.

The heterocyclylthio group may suitably be represented by the formula:

$$- S - Het$$

wherein 'Het' is a five or six membered heterocyclic

ring containing from 1 to 4 atoms selected from N, O, and S unsubstituted or substituted with one or two groups selected from $C_{1-6}$ alkyl, $C_{1-6}$ alkoxy, hydroxyalkyl, $C_{1-6}$ alkenyl, alkoxyalkyl, sulphonylalkyl, carbamoylalkyl, trifluoromethyl, hydroxy, halogen, oxo, aminoalkyl, or substituted aminoalkyl and carboxy- alkyl or two substituents may be linked to form the residue of a heterocyclic or carbocyclic ring.

Examples of the group 'Het' include unsubstituted and substituted imidazolyl, triazolyl, tetrazolyl, thiazolyl, thiadiazolyl, thiatriazolyl, oxazolyl, triazinyl and oxadiazolyl.

Suitable groups 'Het' include unsubstituted and substituted 1, 2, 3-triazolyl; 1, 2, 4-triazolyl; tetrazolyl; oxazolyl; thiazolyl; 1, 3, 4-oxadiazolyl; 1, 3, 4-thiadiazolyl, or 1, 2, 4-thiadiazolyl. Preferably the heterocyclylthio group is 1-methyl-1*H*-tetrazol-5-ylthio, 2-methyl-1,3,4-thia-diazol-5-ylthio, 1-carboxymethyl-1*H*-tetrazol-5-ylthio or 6-hydroxy-2-methyl-5-oxo-2*H*-1,2,4-triazin-3-ylthio.

The nitrogen containing heterocyclic group bonded *via* nitrogen is suitably a pyridinium group unsubstituted or substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylmethyl, carbamoyl, trifluoromethyl, hydroxy, halogen, oxo, aminoalkyl or two substitutents may be linked to form the residue of a carbocyclic ring.

The group R as used herein suitably represents methylthio, phenylthio, tolylthio,methoxy, amino hydroxymethyl, or formamido. Preferably R represents

methylthio or formamido.

Suitably the group $R^1$ represents phenyl, phenyl substituted with up to three groups selected from hydroxy, $C_{1-6}$ alkylcarbonyloxy, $C_{1-6}$ alkoxycarbonyloxy and halogen; cyclohexenyl, cyclohexadienyl or a 5- or 6-membered heterocyclic ring containing up to three hetero-atoms selected from oxygen, sulphur or nitrogen, optionally substituted with hydroxy, amino, halogen, substituted amino or $C_{1-6}$ alkoxy.

Preferably the group $R^1$ is phenyl, 4-hydroxyphenyl, 3,4-di($C_{1-6}$alkylcarbonyloxy)phenyl, 3,4-dibenzyloxycarbonyloxyphenyl, 3,4-dihydroxy-phenyl, 2-thienyl, 3-thienyl or 2-amino-4-thiazolyl.

Particularly preferred groups $R^1$ are 3,4-dihydroxy-phenyl, 3,4-diacetoxyphenyl and 3,4-dibenzyloxy carbonyloxyphenyl.

Suitable $C_{1-6}$ alkyl groups for the groups $R^2$, $R^3$ and $R^4$ in formulae (I), (II) and (III) include methyl, ethyl, n- and iso-propyl, n, sec-, iso- and tert-butyl. Preferably $R^4$ is ethyl. Preferably $R^2$ and $R^3$ are hydrogen.

The group $R^5$ is suitably a hydrocarbon group, in particular $C_{1-6}$ alkyl, aryl, and aryl ($C_{1-6}$)alkyl. In particular, $R^5$ may represent methyl, ethyl, phenyl, benzyl and p-nitrobenzyl.

The cyclisation process of this invention is suitably carried out in a solvent, in the presence of a base. Suitable solvents include water, $C_{1-6}$ alkanols, such as methanol and ethanol, ethers such as tetrahydrofuran and dioxan, esters such as methyl acetate and ethyl

acetate, chlorinated hydrocarbons such as dichloromethane and chloroform, aromatic hydrocarbons such as benzene and toluene, and amides such as dimethylformamide.

The base which is utilised in the present process may be, for example, an alkali metal hydroxide, carbonate, hydride or alkoxide; or a secondary or tertiary amine such as trialkylamine or dialkylbenzylamine or a heterocyclic amine such as pyridine or morpholine optionally substituted with an alkyl or alkylsubstituted amino group. Such amines include 4-dimethylamino-pyridine and N-methylmorpholine. Preferably the base is a strong non-nucleophilic base such as diazabicycloundecene, diazabicyclononene and diazabicyclooctane. A preferred base is 1,8-diazabicyclo[5.4.0]undec-7-ene.

The reaction may be carried out at a temperature, dependent on the chosen solvent, from -20°C to +50°C, suitably from 0°C to 35°C, preferably from 15°C to 20°C.

In the above process, any reactive substituents may be blocked by conventional blocking groups and removed at the end of the cyclisation reaction.

When the compound resulting from the cyclisation process is a $\Delta^2$-cephem it can advantageously be converted to a $\Delta^3$-cephem sulphoxide, by oxidation with a conventional sulphoxide-forming reagent such as a peracid or periodate and, if necessary, rearranging the double bond with a base such as a tertiary amine or alkali metal hydroxide. The $\Delta^3$-cephem sulphoxide may

then be reduced to a $\Delta^3$-cephem, by conventional methods, for example those described in British Patent Specification 1,280,693.

The intermediates of formula (II) are novel compounds and form a further aspect of this invention. A particular subclass of novel intermediates has the formula (IV):

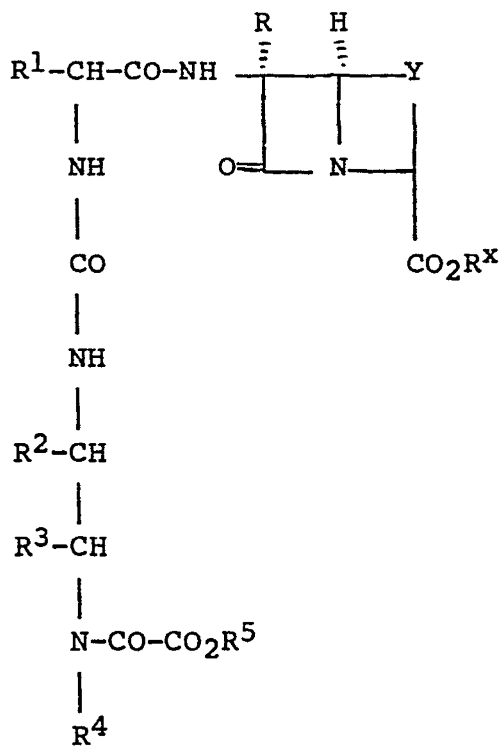

(IV)

wherein R, $R^1$, $R^2$, $R^3$, $R^4$ and $R^5$ are as defined with respect for formula (II) and $R^x$ and Y are as defined with respect to formula (III).

0167011

Compounds of formula (II) may be prepared by a process which comprises reacting a compound of partial stucture (V):

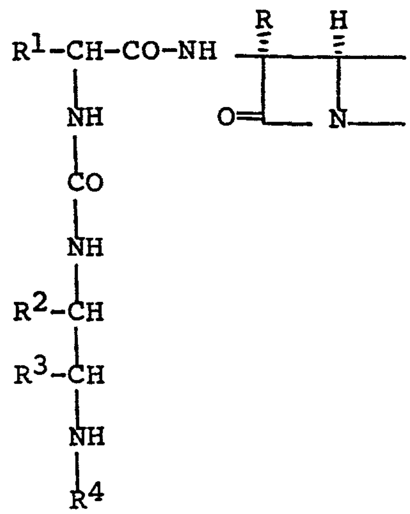

(V)

wherein R, $R^1$, $R^2$, $R^3$ and $R^4$ are as defined with respect to formula (I); with a reactive N-acylating derivative of an acid of formula (VI):

$$R^5O_2C.CO_2H \qquad (VI)$$

wherein $R^5$ is as defined with respect to formula (II).

Thus preferred compounds of formula (IV) may be prepared by a process which comprises reacting a compound of partial structure (Va):

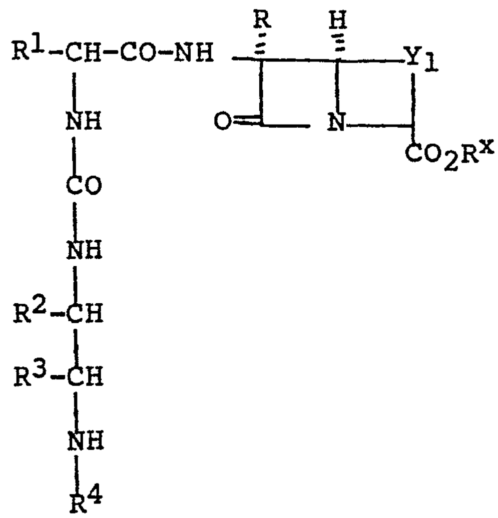

(Va)

wherein R, $R^1$, $R^2$, $R^3$ and $R^4$ are as defined with respect to formula (I) and $R^x$ and Y are as defined with respect to formula III; with a reactive N-acylating derivative of an acid of formula (VI):

$R^5O_2C.CO_2H$ (VI)

wherein $R^5$ is as defined with respect to formula (II).

Suitable N-acylating derivatives include an acid halide, preferably the acid chloride or bromide. Acylation with an acid halide may be effected in the presence of an acid binding agent for example, tertiary amine (such as triethylamine or dimethylaniline), an inorganic base (such as calcium carbonate or sodium bicarbonate) or an oxirane, which binds hydrogen halide

liberated in the acylation reaction. The oxirane is preferably a $(C_{1-6})$-1,2-alkylene oxide - such as ethylene oxide or propylene oxide. The acylation reaction using an acid halide may be carried out at a temperature in the range -50°C to +50°C, preferably -20°C to +20°C, in aqueous or non-aqueous media such as water, tetrahydrofuran, dimethylacetamide, dimethylformamide, acetonitrile, dichloromethane, chloroform, 1,2-dichloroethane, an aliphatic ester such as ethyl acetate or butyl acetate, an aliphatic ketone such as acetone or 4-methylpentan-2-one, or mixtures thereof.

Alternatively, the N-acylating derivative of the acid (VI) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as p-toluenesulphonic acid). When a symmetrical anhydride is employed, the reaction may be carried out in the presence of 2,6-lutidine as catalyst.

Alternative N-acylating derivatives of acid (VI) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, or 8-hydroxyquinoline; or amides such as N-acylsaccharins, N-acylthiazolidin-2-thione or N-acylphthalimides; or an alkylidene iminoester prepared by reaction of the acid (VI) with an oxime.

Other reactive N-acylating derivatives of the acid (VI) include the reactive intermediates formed by reaction in situ with a condensing agent such as a carbodiimide, for example, N,N'-diethyl-, dipropyl- or diisopropylcarbodiimide, N,N'-di-cyclohexyl-carbodiimide, or N-ethyl-N' [3-(dimethylamino)propyl]-carbodiimide; a suitable carbonyl compound, for example, N,N'-carbonyldiimidazole or N,N'-carbonylditriazole; an isoxazolinium salt, for example, N-ethyl-5-phenylisoxolinium-3-sulphonate or N-7-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

The compound of formula (V) or (Va) may be prepared by reacting a compound of partial structure (VII):

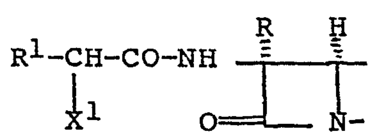

(VII)

or a compound of formula (VIIa)

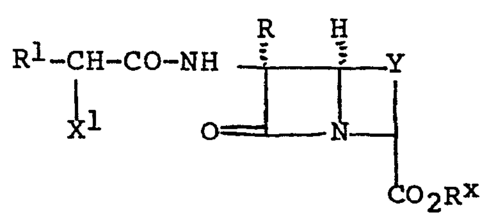

(VIIa)

wherein R and $R^1$ are as defined with respect to formula (I) and Y and $R^x$ are as defined with respect to formula (III); with a compound of formula (VIII)

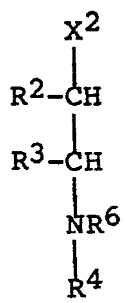

(VIII)

wherein $R^2$, $R^3$ and $R^4$ are as defined with respect to formula (I) and $R^6$ is a removable amino protecting group, one of $X^1$ and $X^2$ represents amino, and the other represents an N-acylating derivative of a carbamic acid group, $-NH.CO_2H$; and thereafter removing protecting group $R^6$.

Examples of N-protecting groups in $R^6$ include $C_{1-6}$ alkanoyl, for example acetyl, propionyl n- and isobutyryl and 2,2-dimethylpropanoyl, benzoyl or benzene optionally substituted in the phenyl ring by one or two substituents selected from $C_{1-4}$ alkyl, $C_{1-4}$ alkoxy, trifluoromethyl, halogen or nitro; and $C_{1-4}$ alkoxycarbonyl, for example tert-butoxycarbonyl or 2,2,2-trichloroethoxycarbonyl, or benzyl optionally substituted as for benzoyl above.

Particular examples of $R^6$ protecting groups include those listed above for N-protecting groups which are hydrogenolysable.

Removal of the protecting group $R^6$ may be effected by conventional methods. For example, when $R^6$ is $C_{1-6}$ alkanoyl or optionally substituted benzoyl as defined, removal is conveniently effected by conventional base hydrolysis.

When $R^6$ is $C_{1-4}$ alkoxycarbonyl or optionally substituted benzyloxycarbonyl as defined, removal may be carried out by hydrogenolysis. Suitable reactions are conventional transition-metal catalysed hydrogenation, using for example palladium-, or platinum-, charcoal, at atmospheric pressure or a slight excess thereover. An inert polar solvent such as ethanol and ambient temperatures are apt.

When the group $X^1$ or $X^2$ represents an N-acylating derivative of a group $-NH.CO_2H$, a preferred N-acylating derivative is the isocyanate group -N=C=O.

Other suitable N-acylating derivatives include the acid halide, preferably the acid chloride, -NH.COCl, or activated esters, such as esters with 2-mercaptopyridine, cyanomethanol, trichloromethanol, p-nitrophenol, 2,4-dinitrophenol, thiophenol, halophenols, including pentachlorophenol, monomethoxyphenol, N-hydroxy succinimide, or 8-hydroxyquinoline; or amides formed from amines such as saccharins, thiazolidin-2-thione, phthalimides, imidazole or triazole.

A preferred activated ester is the p-nitrophenyl ester, $-NH.CO_2C_6H_4NO_2$, and a preferred thiol ester is the thiophenol ester, $-NHCOSC_6H_5$.

The free carbamic acid, i.e. a compound where $X^1$ or $X^2$ is $-NH.CO_2H$, is not generally sufficiently stable to isolate.

The compounds of formulae (VII), (VIIa) and (VIII) wherein $X^1$ or $X^2$ is an N-acylating derivative of a group $-NH.CO_2H$ may be prepared from the corresponding compound where $X^1$ or $X^2$ represents amino, by

conventional methods. For example the preferred N-acylating derivative, the isocyanate, where $X^1$ or $X^2$ is -N=C=O, may be prepared by reacting the compound wherein $X^1$ or $X^2$ is amino, with phosgene.

The acid chloride, where $X^1$ or $X^2$ is -NH.COCl, may be generated from the amino compound by reaction with phosgene, and employed <u>in situ</u>. The activated esters may be prepared from the corresponding chloroformate derivative of the alcohol, phenol or thiophenol. The activated amides may be prepared from the corresponding carbonyl compound for example N,$N^1$-carbonyldiimidazole or N,N'-carbonylditriazole.

In the reaction between compound (VII) or (VIIa) and compound (VIII), preferably $X^1$ represents amino and $X^2$ represents an N-acylating derivative, in particular the isocyanate group of formula -N=C=O.

Alternatively, the novel intermediates of formula (II) or (IV) may be prepared by reacting a compound of formula (VII) or (VIIa) with a compound of formula (IX):

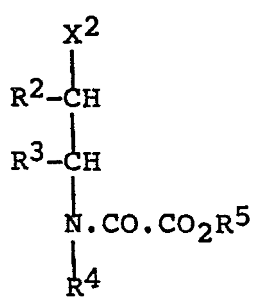

(IX)

wherein $R^2$, $R^3$, $R^4$, $R^5$, and $X^2$ are as defined above.

The compounds of formula (IX) are also novel compounds, both when $X^2$ represents amino and when $X^2$ represents an N-acylating derivative of the group $-NH.CO_2H$, and form a further aspect of this invention. Those compounds wherein $X^2$ represents an N-acylating derivative may be prepared from the compound wherein $X^2$ represents amino by the methods described above.

The compounds of formulae (VIII) and (IX) which have the most utility as intermediates are compounds (VIII) where $X^2$ represents an N-acylating derivative of the group $-NH.CO_2H$, and compounds (IX) where $X^2$ represents either amino or an N-acylating derivative of a group $-NH.CO_2H$.

The compounds of formula (IX) where $X^2$ represents amino may be prepared by reacting a compound of formula (X)

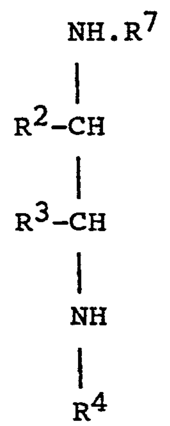

(X)

wherein $R^2$, $R^3$ and $R^4$ are as defined above, and $R^7$ is an amino protecting group; with a reactive N-acylating derivative of an acid of formula (VI) as described above; and subsequently removing the amino protecting group.

Suitable amino-protecting groups $R^7$ are those well-known in the art which may be removed under conventional conditions without disruption of the remainder of the molecule.

Examples of suitable groups $R^7$ which may subsequently be converted to $-NH_2$ by mild acid hydrolysis include groups of formula (XI) or tautomeric modifications thereof:

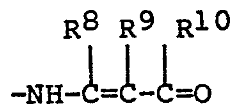

(XI)

wherein $R^8$ is $C_{1-6}$ alkyl, $R^9$ is hydrogen or $C_{1-6}$ alkyl, $R^{10}$ is $C_{1-6}$ alkyl, aryl or $C_{1-6}$ alkoxy; or $R^9$ together with either $R^8$ or $R^{10}$ completes a carbocyclic ring.

Preferably $R^8$ is methyl, $R^9$ is hydrogen and $R^{10}$ is methoxy.

Compounds of formula (VIII), where $X^2$ is amino, may be prepared by protecting compound (X) with an amino protecting group $R^6$, and subsequently removing the protecting group $R^7$.

Compounds of formula (X), may be prepared by amino protection of the compound of formula (XII):

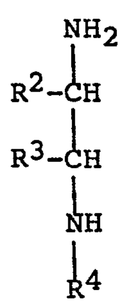

(XII)

Compounds of formula (II) or of formula (IV) may also be prepared by reacting a compound of formula (XIII):

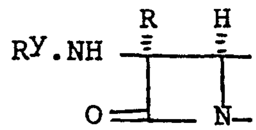

(XIII)

or a compound of formula (XIIIa)

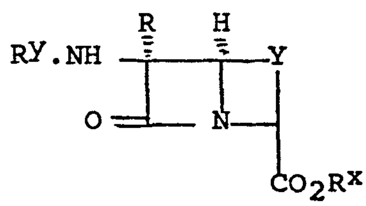

(XIIIa)

where R is as defined above, Y and $R^x$ are as defined with respect to formula (III) and $R^y$ represents hydrogen or a group which permits acylation to take place; with a reactive N-acylating derivative of a compound of formula (XIV):

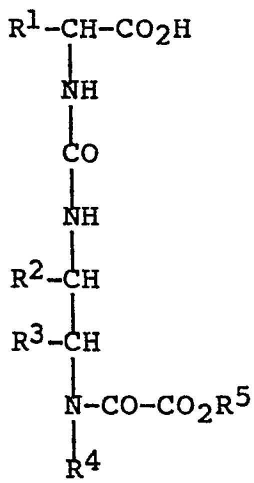

(XIV)

wherein $R^1$, $R^2$, $R^3$, $R^4$ and $R^5$ are as defined above.

Suitable reactive N-acylating derivatives of the acid (XIV) are as described above with respect to the acid (VI).

Suitable groups which permit acylation to take place and which are optionally present on the amino group of the starting material of the formula (XIII) or (XIIIa) include N-silyl, N-stannyl and N-phosphorus groups, for example trialkylsilyl groups such as trimethylsilyl, trialkyltin groups such as tri-n-butyltin, groups of formula $-P.R^aR^b$
wherein
$R^a$ is an alkyl, haloalkyl, aryl, aralkyl, alkoxy, haloalkyl, aryl, aralkyl, alkoxy, haloalkoxy, aryloxy, aralkyloxy or dialkylamino group,
$R^b$ is the same as $R^a$ or is halogen or
$R^a$ and $R^b$ together form a ring; suitable such phosphorus groups being $-P(OC_2H_5)_2$, $-P(C_2H_5)_2$,
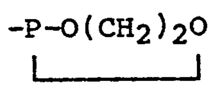 and 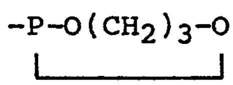

Compounds of formula (XIV) are generally described in Japanese patent application no. 112605/77 (Kokai 46799/79). However, in a further aspect of the present invention, there is provided an improved process for the preparation of a compound of formula (XIV), which process comprises reaction of a compound of formula (XV):

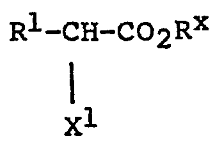

(XV)

wherein $R^1$, $R^x$ and $X^1$ are as hereinbefore defined; with a compound of formula (IX) as hereinbefore defined; and thereafter if necessary removing any blocking group $R^x$.

The comments above relating to $R^x$, $X^1$ and $X^2$ also relate to this process.

A further novel process for the preparation of compounds of formula (XIV) comprises reacting a compound of formula (XVI):

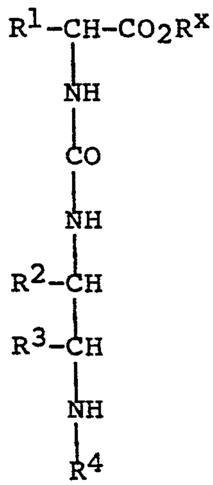

(XVI)

wherein $R^1$, $R^2$, $R^3$, $R^4$ and $R^x$ are as hereinbefore defined; with a reactive N-acylating derivative of an acid of formula (VI):

$$R^5O_2C.CO_2H \qquad (VI)$$

as hereinbefore defined; and thereafter if necessary removing any blocking group $R^x$.

Furthermore the present invention also comprises a class of compounds within the general definition of formula (XIV), which are not disclosed in Japanese application 112605/77, namely compounds of formula (XIV) wherein $R^1$ represents a dihydroxyphenyl or protected dihydroxyphenyl group.

Within compounds (XIV), preferably $R^1$ represents 3,4-dihydroxyphenyl, 3,4-diacetoxyphenyl, 3,4-diethoxycarbonyloxyphenyl or 3,4-dibenzyloxycarbonyloxyphenyl. Preferably R`represents 3,4-dihydroxyphenyl.

Preferably $R^2$ and $R^3$ represent hydrogen.

Preferably $R^4$ represents $C_{1-6}$ alkyl, in particular ethyl.

The following Examples illustrate the present invention.

Preparation 1

4-Nitrobenzyl [N-(2-isocyanatoethyl)-N-ethylamino]oxalate

(i) Methyl 3-[2-(ethylamino)ethylamino]but-2-enoate

A solution of N-ethylethylenediamine (5.3 ml, 4.4 g, 50 mmol) and methyl acetoacetate (5.4 ml, 5.8 g, 50 mmol) in dichloromethane (125 ml) was refluxed under argon for 4 h, then filtered through a short plug of silica, and evaporated to dryness giving the product as a colourless oil (9.25 g, 99.5%), $\nu_{max.}$ ($CHCl_3$) 3300 br, 2950, 1645, 1605, 1550, 1500, 1440, and 1380 $cm^{-1}$; $\delta$(90 MHz, $CDCl_3$) 1.10 (3H, t, J 7.5 Hz, $NCH_2C\underline{H}_3$), 1.40 (1H, s, $N\underline{H}CH_2CH_3$), 1.90 (3H, s, $CH_3C=C$), 2.62 (2H, t, J 7.5 Hz, $C\underline{H}_2NCH_2CH_3$), 2.77 (2H, t, J 7.5 Hz, $CH_2NC=C$), 3.30 (2H, q, J 7.5 Hz, $NC\underline{H}_2CH_3$), 3.57 (3H, s, $CO_2CH_3$), 4.43 (1H, s, C=CH), and 8.60 (1H, br s, NHC=C); (Found: $M^+$, 186.1367; $C_9H_{18}N_2O_2$ requires M, 186.1368).

(ii) Methyl 3-[2-[N-ethyl-N-(4-nitrobenzyloxalyl)amino]ethylamino]but-2-enoate

4-Nitrobenzyloxalyl chloride (2.44 g, 0.01 mol) in dichloromethane (15 ml) was added dropwise with stirring to a solution of methyl 3-[2-(ethylamino)ethylamino]but-2-enoate (1.86 g, 0.01 mol) and N,N-diisopropylethylamine (1.29 g, 0.01 mol) in dichloromethane (20 ml) at 0°C. The solution was allowed to warm slowly to room temperature over 3 h and it was then evaporated to dryness, and the residue quickly chromatographed on silica gel 60 eluting with hexane/ethyl acetate 2:3 to afford the essentially pure product (2.60 g, 66%) as a yellow oil; $\nu_{max.}$ ($CH_2Cl_2$) 1745, 1660, 1610, 1525, 1350 $cm^{-1}$; n.m.r. showed two isomers, ratio 2:1, $\delta$($CDCl_3$) 1.15 and 1.17 (3H, 2t, J 7Hz, 2 $CH_2C\underline{H}_3$), 1.83 and 1.91 (3H, 2s, $CH_3C=C$), 3.10 -

3.70 (9H, m, 3 $NCH_2$ and $CH_3O_2C$), 4.46 (1H, s, C=C*H*), 5.35 (2H, s, C*H*$_2$Ar), 7.54 and 8.18 (4H, AA'BB', *J* 9Hz, aromatics) and 8.55 (1H, br s, NH); (Found *M*$^+$, 393.1545. $C_{18}H_{23}N_3O_7$ requires *M*, 393.1536).

(iii) 2-[*N*-Ethyl-*N*-(4-nitrobenzyloxalyl)amino]ethylammonium chloride

A solution of methyl 3-[2-[*N*-ethyl-*N*-(4-nitrobenzyloxalyl)amino]ethylamino]but-2-enoate (1.35 g, 3.44 mmol) in acetone (13 ml) was treated with 5 M hydrochloric acid (0.7 ml, 3.5 mmol) and the solution was allowed to stand at room temperature for 0.5 h. It was then cooled in an ice bath and treated with ether (*ca.* 5 ml), which caused immediate crystallisation. The solid was filtered off, washed with diethyl ether and then dried to afford the title compound (0.71 g, 63%); m.p. 157 - 9°C; $\nu_{max.}$ (KBr) 3300 - 2400 v. br., 1742, 1659, 1526, 1342, 1223, 1147 $cm^{-1}$; $\delta(D_2O)$ 1.30 (3H, t, *J* 7Hz, $NCH_2CH_3$), 3.30 - 3.70 (4H, m, $NCH_2CH_3$ and $NCH_2$), 3.88 (2H, t, *J* 6Hz, $NCH_2$), 5.59 (2H, s, C*H*$_2$Ar), 7.74 and 8.31 (4H, AA'BB', *J* 8Hz, aromatics); (Found: C, 47.3; H, 5.5; N, 12.7%; *M*$^+$ - $H_2O$, 277.1058. $C_{13}H_{18}ClN_3O_5$ requires C, 47.1; H, 5.5; N, 12.7%; *M* - $H_2O$, 277.1062).

(iv) 4-Nitrobenzyl [*N*-(2-isocyanatoethyl)-*N*-ethylamino] oxalate

2-[*N*-Ethyl-*N*-(4-nitrobenzyloxalyl)amino]ethylammonium chloride (166 mg, 0.5 mmol) was suspended in dichloromethane (7 ml) and the mixture was cooled to 0°C. Triethylamine (152 mg, 1.5 mmol) was then added and the resulting solution was treated with a 12.5% w/w solution of phosgene in toluene (435 µl, 0.5 mmol). The reaction solution was stirred at 0°C for 5 min followed

by 1 h at room temperature, and it was then evaporated to dryness. The residue was treated with tetrahydrofuran and the insoluble triethylamine hydrochloride was filtered off and dried (190 mg, 92%). The filtrate was evaporated to afford the title isocyanate; $\nu_{max.}$ ($CH_2Cl_2$) 2275, 1745, 1720, 1660, 1530, 1350 $cm^{-1}$.

Preparation 2

2-[N-Ethyl-N-(2,2,2-trichloroethoxycarbonyl)amino]ethylammonium chloride

(i) Methyl 3-[2-[N-ethyl-N-(2,2,2-trichloroethoxycarbonyl)amino]ethylamino]but-2-enoate

Methyl 3-[2-(ethylamino)ethylamino]but-2-enoate (1.86 g, 10 mmol) was dissolved in anhydrous dichloromethane (35 ml) containing triethylamine (1.5 ml, 10.5 mmol) and cooled to 0°C with stirring. 2,2,2-Trichloroethoxycarbonyl chloride (1.40 ml, 10.5 mmol) was added in one portion; precipitation of white solid was soon observed. The mixture was allowed to regain room temperature and stirred for a further 0.5 h, after which it was poured into ethyl acetate (100 ml) and washed successively with 0.5 M hydrochloric acid (2 x 50 ml), saturated aqueous sodium hydrogen carbonate solution (50 ml), water and brine. The final extract was dried over anhydrous magnesium sulphate and evaporated to give the title compound as a pale yellow oil (3.14 g, 87%); δ($CDCl_3$) 1.2 (3H, t, $C\underline{H}_3CH_2N$), 1.9 (3H, s, $C\underline{H}_3C{=}C$), 3.0 - 3.7 (9H, m, 3 x $NCH_2$ + $OCH_3$), 4.4 (1H, s, $C\underline{H}{=}C$), 4.7 (2H, s, $Cl_3CC\underline{H}_2O$), 8.5 (1H, br s, NH); $R_F$ 0.80 in 10% methanol-chloroform.

(ii) 2-[N-Ethyl-N-(2,2,2-trichloroethoxycarbonyl)amino] ethylammonium chloride

Methyl 3-[2-(N-ethyl-N-(2,2,2-trichloroethoxycarbonyl)amino]ethylamino]but-2-enoate (2.66 g, 7.36 mmol) in acetone (10 ml) was treated with 2 M hydrochloric acid (3.7 ml). After 0.5 h, when no starting material was visible by t.l.c., the solution was diluted to 200 ml with ether, containing a little more acetone to prevent the separation of an aqueous phase. The precipitated

solid was filtered, well washed with ether, dried and recrystallised from methanol-ether to afford the amine hydrochloride (1.58 g, 63%), m.p. 157-158°C; $\nu_{max.}$ (KBr) 1699, 1591, 1491, 1473, 1452, 1431 $cm^{-1}$; $\delta$[$(CD_3)_2SO$] 1.11 (3H, t, $C\underline{H}_3CH_2N$), 2.8 - 3.6 (6H, m, 3 $NCH_2$), 4.79 (2H, s, $Cl_3CCH_2O$), 8.27 (3H, br s, $D_2O$ exchanged, $\overset{+}{N}H_3$) (Found: C, 28.0; H, 4.7; Cl, 47.2; N, 9.3. $C_7H_{14}Cl_4N_2O_2$ requires C, 28.0; H, 4.7; Cl, 47.3; N, 9.3%).

Example 1

Benzyl 6β-[D-2[[2-[N-ethyl-N-(4-nitrobenzyloxalyl)amino]-ethylamino]carbonylamino]-2-phenylacetamido]-6α-(methylthio)penicillanate (method (i))

2-[N-Ethyl-N-(4-nitrobenzyloxalyl)amino]ethylammonium chloride (166 mg, 0.5 mmol) was suspended in dichloromethane (7 ml), cooled to 0°C and treated with triethylamine (253 mg, 2.5 mmol). The resulting clear solution was treated in one portion with a 12.5% w/w solution of phosgene in toluene (435 μl, 0.5 mmol), and it was then allowed to warm to room temperature over 1 h. The reaction solution was then cooled to 0°C and a solution of benzyl 6β-[D-2-amino-2-phenylacetamido]-6α-(methylthio)penicillanate (243 mg, 0.5 mmol) in dichloromethane (5 ml) was added dropwise over approximately 1 min. The solution was stirred at 0°C for 5 min followed by 2.5 h at room temperature, before being evaporated to small volume, redissolved in ethyl acetate (40 ml) and washed with 0.5 M hydrochloric acid and brine. The organic solution was dried over $MgSO_4$, filtered and evaporated to leave the crude product which was chromatographed on silica gel 60 (20 g) eluting with hexane/ethyl acetate 1:2 grading to 2:5 to afford the title compound (142 mg, 35%); $\nu_{max.}$ ($CH_2Cl_2$) 3380, 1785, 1745, 1655, 1525, 1350 $cm^{-1}$; n.m.r. showed the presence of two isomers, δ($CDCl_3$) 0.92 and 0.96 (3H, 2s, 2-$CH_3$), 1.00 - 1.23 (6H, m, 2-$CH_3$ and $CH_2CH_3$), 2.28 (3H, s, $SCH_3$), 3.13 - 3.63 (6H, m, 3 $NCH_2$), 4.32 (1H, s, 3-H), 5.11 and 5.17 (2H, ABq, J 12Hz, $CH_2Ph$), 5.19 and 5.28 (1H, ABq, J 14Hz, $CH_2$PNB isomer), 5.34 (1H, s, $CH_2$PNB isomer), 5.46 and 5.47 (1H, 2s, 5-H isomers), 5.78 (1H, m, $CH_2NHCO$) 5.88 and 5.98 (1H, 2d, J 8Hz, CHNHCO isomers), 6.88 and 7.03 (1H, 2d, J 8Hz, CHNHCO), 7.10 - 7.40 (8H, m, aromatics), 7.40 - 7.60

(4H, m, aromatics), 8.13 and 8.22 (2H, AA'BB', *J* 8Hz, PNB aromatics isomers), 8.29 and 8.50 (1H, 2s, 6β-NHCO); (Found *MH*$^+$, 807. $C_{38}H_{42}N_6O_{10}S_2$ requires *M*, 806).

Example 2

Benzyl 6β-[D-2[[2-[N-ethyl-N-(4-nitrobenzyloxalyl)amino]-ethylamino]carbonylamino]-2-phenylacetamido]-6α-(methylthio)penicillanate (method (ii))

4-Nitrobenzyl [N-(2-isocyanatoethyl)-N-ethylamino] oxalate (0.25 mmol) in dichloromethane (3 ml) at 0°C was treated with a solution of benzyl 6β-[D-2-amino-2-phenylacetamido]-6α-(methylthio)penicillanate (122 mg, 0.25 mmol) in dichloromethane (3 ml), and the reaction solution was stirred at 0°C for 0.5 h followed by 5 h at room temperature. The solution was then evaporated to dryness and the crude product was chromatographed on silica gel 60 eluting with hexane/ethyl acetate 1:3 grading to 1:4 to afford the title compound (65 mg, 32%).

0167011

Example 3

Benzyl 6β-[D-2[(4-ethyl-2,3-dioxopiperazin-1-yl)carbonylamino]-2-phenylacetamido]-6α-(methylthio)penicillanate

A 0.01 M solution of 1,8-diazabicyclo[5.4.0]undec-7-ene in dichloromethane (7.4 ml, 0.074 mmol) was added to a solution of benzyl 6β-[D-2-[[2-[N-ethyl-N-(4-nitrobenzyloxalyl)amino]ethylamino]carbonylamino]-2-phenylacetamido]-6α-(methylthio)penicillanate (60 mg, 0.074 mmol) in dichloromethane (6 ml), and the reaction solution was stirred at room temperature for 15 min. It was then washed with 0.25 M hydrochloric acid (2 x 10 ml) and brine, before being evaporated to dryness. The residue was chromatographed on silica gel 60 eluting with ethyl acetate to afford the title compound (29 mg, 60%); $\nu_{max.}$ ($CH_2Cl_2$) 3390, 3300, 1780, 1742, 1718, 1690 $cm^{-1}$; $\delta$($CDCl_3$) 0.96 (3H, s, 2-$CH_3$), 1.21 (6H, m, 2-$CH_3$ and $CH_2C\underline{H}_3$) 2.28 (3H, s, $SCH_3$), 3.40 - 3.70 (4H, m, $C\underline{H}_2CH_3$ and piperazine $CH_2$), 3.99 (1H, m, piperazine CH), 4.18 (1H, m, piperazine CH), 4.32 (1H, s, 3-H), 5.12 and 5.19 (2H, ABq, $\underline{J}$ 12Hz, $C\underline{H}_2Ph$), 5.53 (1H, s, 5-H), 5.60 (1H, d, $\underline{J}$ 7Hz, $NHC\underline{H}$), 7.07 (1H, s, 6β-NHCO), 7.25 - 7.55 (10H, m, aromatics), and 10.04 (1H, d, $\underline{J}$ 7Hz, $N\underline{H}CH$); (Found $\underline{MH}^+$, 654. $C_{31}H_{35}N_5O_7S_2$ requires $\underline{M}$, 653).

Example 4

Benzyl 6β-[D-2-[[2-[N-ethyl-N-(4-nitrobenzyloxalyl)-amino]ethylamino]carbonylamino]-2-(3,4-diacetoxyphenyl)-acetamido]-6α-formamidopenicillanate

Benzyl 6β-[D-2-(2,2,2-trichloroethoxycarbonylamino)-2-(3,4-diacetoxyphenyl)acetamido]-6α-formamidopenicillanate (630 mg, 0.814 mmol) in tetrahydrofuran (15 ml) was treated with 1 M potassium dihydrogen phosphate (3 ml) and freshly acid washed zinc (1.0 g). The reaction mixture was stirred at pH 4 for 1 h before addition of more zinc (1.0 g). Reaction was continued at room temperature until complete by t.l.c. (total of 2.5 h). The reaction mixture was filtered and the filtrate evaporated to small volume and diluted with ethyl acetate. The mixture was washed with brine, dried over $MgSO_4$ and then evaporated to leave benzyl 6β-[D-2-amino-2-(3,4-diacetoxyphenyl)acetamido]-6α-formamidopenicillanate. This, in dichloromethane (10 ml), was then added dropwise to a solution of 4-nitrobenzyl [N-(2-isocyanatoethyl)-N-ethylamino]oxalate (0.814 mmol) in dichloromethane (10 ml) at 0°C. The reaction solution was stirred at 0°C for 0.5 h followed by 2 h at room temperature, and then it was evaporated to dryness. Chromatography of the residue on silica gel 60 eluting with 2% grading to 5% ethanol/chloroform afforded the title compound (88 mg, 12%); $\nu_{max.}$ ($CH_2Cl_2$) 3305, 1780, 1745, 1690, 1660, 1525, 1350, 1210, 1185 $cm^{-1}$; n.m.r. showed two major isomers, δ[$(CD_3)_2CO$] 1.03 (3H, s, 2-$CH_3$), 1.10 and 1.11 (3H, 2t, J 7Hz, 2 $C\underline{H}_3CH_2$), 1.22 (3H, s, 2-$CH_3$), 2.24 (6H, s, 2 $CH_3CO_2$), 3.20 - 3.57 (6H, m, 3 $NCH_2$), 4.41 and 4.42 (1H, 3s, 3-H), 5.19 (2H, s, $C\underline{H}_2Ph$), 5.41 (1H, s, 5-H), 5.49 and 5.58 (2H, 2s, $C\underline{H}_2PNB$ isomers), 5.73 (1H, d, J 7Hz, ArC$\underline{H}$NH), 6.10 (1H, m, $CH_2N\underline{H}CO$), 6.65 (1H, m, ArCHN$\underline{H}$), 7.15

(1H, d, *J* 8Hz, aromatics), 7.30 - 7.50 (7H, m, aromatics), 7.73 (2H, m, PNB aromatics), 8.16 (1H, s, NHC*H*O), 8.20 - 8.35 (3H, m, PNB aromatics), 8.89 and 9.12 (1H, s and d, N*H*CHO). (Found *M*H$^+$, 920. $C_{42}H_{45}N_7O_{15}S$ requires *M*, 919).

Example 5

Benzyl 6β-[D-2-[(4-ethyl-2,3-dioxopiperazin-1-yl)-carbonylamino]-2-(3,4-diacetoxyphenyl)acetamido]-6α-formamidopenicillanate

A solution of benzyl 6β-[D-2-[[2-[N-ethyl-N-(4-nitrobenzyloxalyl)amino]ethylamino]carbonylamino]-2-(3,4-diacetoxyphenyl)acetamido]-6α-formamidopenicillanate (57 mg, 0.062 mmol) in dichloromethane (10 ml) was treated with a solution of 1,8-diazabicyclo[5.4.0]undec-7-ene (0.062 mmol) in dichloromethane (1.24 ml) and the reaction solution was stirred at room temperature for 15 min. It was then washed with 0.5 M hydrochloric acid and brine, and then dried over $MgSO_4$. After evaporation to dryness, the crude product was chromatographed on silic gel 60 eluting with ethyl acetate grading to 3% ethanol/ethyl acetate to afford the title compound (22 mg, 46%); $\nu_{max.}$ ($CH_2Cl_2$) 2275, 1780, 1745, 1710 sh, 1690, 1500, 1210, 1185 $cm^{-1}$; n.m.r. showed two rotamers, δ[$(CD_3)_2CO$] 0.97 (3H, s, 2-$CH_3$), 1.17 (3H, t, J 7Hz, $CH_2CH_3$), 1.22 (3H, s, 2-$CH_3$), 2.25 and 2.27 (6H, 2s, 2 x $CH_3CO_2$), 3.51 (2H, q, J 7Hz, $CH_2CH_3$), 3.67 (2H, m, piperazine $CH_2$), 4.01 (2H, m, piperazine $CH_2$), 4.39 and 4.48 (1H, 2s, 3-H rotamers), 5.19 (2H, s, $CH_2Ph$), 5.58 (1H, s, 5-H), 5.74 (1H, d, J 7Hz, ArCHNH), 7.12 - 7.57 (8H, m, aromatics), 8.18 (1H, s, NHCHO) 8.28 (1H, s, NHCHO), 8.94 and 9.15 (1H, 2s, 6β-NHCO rotamers), 10.09 (1H, d, J 7Hz, ArCHNH). (Found $MH^+$, 767. $C_{35}H_{38}N_6O_{12}S$ requires M, 766).

Example 6

Benzyl 6β-[D-2-[[2-[N-ethyl-N-(2,2,2-trichloroethoxycarbonyl)amino]ethylamino]carbonylamino]-2-phenylacetamido]-6α-(methylthio)penicillanate

2-[N-Ethyl-N-(2,2,2-trichloroethoxycarbonyl)amino]-ethylammonium chloride (0.3 g, 1 mmol) was suspended in anhydrous dichloromethane (10 ml) and treated with triethylamine (0.7 ml, 5 mmol) under argon. The clear solution which soon resulted was cooled to 0°C and stirred, then a solution of phosgene in toluene (12.5% w/w, 0.9 ml, 1 mmol) was added. The mixture was allowed to regain room temperature, then stirred for a further 0.5 h. An aliquot of the mixture, on dilution with anhydrous tetrahydrofuran, filtration and evaporation to dryness showed $\nu_{max.}$ ($CHCl_3$) 2275, 1710 and 1530 $cm^{-1}$; the bulk of the isocyanate was not isolated but immediately reacted as follows. The mixture was again cooled to 0°C and a solution of benzyl 6β-[D-2-amino-2-phenylacetamido]-6α-(methylthio)penicillanate (0.49 g, 1 mmol) in anhydrous dichloromethane (5 ml) was added dropwise. On again warming to room temperature a clear solution resulted; stirring was continued for 0.5 h, then the solution was poured into EtOAc (70 ml) and washed with 0.5 M hydrochloric acid (2 x 30 ml), saturated aqueous sodium hydrogen carbonate (2 x 30 ml), water and brine. The final extract was dried over anhydrous magnesium sulphate, evaporated to dryness, and chromatographed on silica gel 60 (50 g), eluting with 3% methanol in chloroform. Appropriate fractions were pooled and evaporated to afford the title compound (0.59 g, 76%); $\nu_{max.}$ (KBr) 1782, 1745, 1715, 1644, 1546 $cm^{-1}$; δ[$(CD_3)_2CO$] 1.05 - 1.25 [9H, m, $(CH_3)_2C$ and $C\underline{H}_3CH_2N$], 2.32 (3H, s, $CH_3S$), 3.25 - 3.45 (6H, m, 3 $CH_2N$), 4.40 (1H, s, 3-H), 4.81 (2H, ABq, $Cl_3CCH_2O$),

5.21 (2H, ABq, PhC$\underline{H}_2$O), 5.43 (1H, s, 5-H), 5.64 (1H, d, s on $D_2O$ exchange, C$\underline{H}$NH), 6.0 (1H, v br s, $D_2O$ exchanged, NH), 6.43 (1H, t, $D_2O$ exchanged, $CH_2N\underline{H}$), 7.2 - 7.6 (10H, m, ArH), 8.71 (1H, s, $D_2O$ exchanged, 6-NH); $R_f$ 0.50 in 5% methanol-chloroform; (Found: $\underline{MH}^+$, 774. $C_{32}H_{38}Cl_3N_5O_7S_2$ requires $\underline{M}$ 773).

Example 7

Benzyl 6β-[D-2-[(4-ethyl-2,3-dioxopiperazin-1-yl)-carbonylamino]-2-phenylacetamido]-6α-(methylthio)-penicillanate

(i) Benzyl 6β-[D-2-[[2-[N-ethyl-N-(methyloxalyl)amino]-ethylamino]carbonylamino]-2-phenylacetamido]-6α-(methylthio)penicillanate

Benzyl 6β-[D-2-[[2-[N-ethyl-N-(2,2,2-trichloroethoxycarbonyl)amino]ethylamino]carbonylamino]-2-phenyl-acetamido]-6α-(methylthio)penicillanate (380 mg, 0.49 mmol) was dissolved in tetrahydrofuran (15 ml) and 1 M potassium dihydrogen phosphate (3 ml) was added. The mixture was stirred vigorously; freshly acid-washed zinc (0.8 g) was added and the pH maintained around 3.0 - 4.0 by the addition of hydrochloric acid, the course of the reduction being monitored by t.l.c. After 1.5 h a further portion of zinc (0.5 g) was added; a further 0.5 h later, no starting material was visible by t.l.c. The solids were filtered and washed with water and ethyl acetate; the aqueous phase was saturated with sodium chloride, the organic phase was separated and the aqueous phase was extracted twice more with ethyl acetate : tetrahydrofuran, 1:1. The total organic extract was dried over anhydrous magnesium sulphate and evaporated to dryness; the residue was used at once without further purification ($R_f$ ca. 0.1 in 10% methanol-chloroform). It was suspended in dry dichloromethane (5 ml) and treated with triethylamine (0.14 ml, 1 mmol) when a virtually clear solution resulted; this was cooled to 0°C, stirred and treated with methyl oxalyl chloride (0.045 ml, 0.5 mmol). The solution was allowed to regain room temperature, then stirred for a further 0.5 h. Workup for a neutral

product as described in Example 6 gave crude product (148 mg), which was chromatographed on silica gel 60 (16 g) eluting with 3% methanol-chloroform, to afford the title compound (78 mg, 23%); $R_f$ 0.65 in 10% methanol-chloroform; $\nu_{max.}$ (KBr) 1782, 1742, 1653, 1586 w, 1550, 1495 $cm^{-1}$; $\delta[(CD_3)_2CO]$ 1.11, 1.22 [6H, 2s, $(CH_3)_2C$], 1.13 (3H, t, $\underline{CH_3}CH_2N$), 2.32 (3H, s, $CH_3S$), 3.2 - 3.5 (6H, m, 3 $CH_2N$), 3.76 (3H, s, $CH_3O$), 4.41 (1H, s, 3-H), 5.21 (2H, ABq, $PhC\underline{H_2}O$), 5.43 (1H, s, 5-H), 5.64 (1H, d, C$\underline{H}$NH), 6.01 (1H, t, $D_2O$ exchanged, N$\underline{H}$$CH_2$), 6.50 (1H, d, $D_2O$ exchanged, N$\underline{H}$CH), 7.2 - 7.6 (10H, m, ArH), 8.72 (1H, s, $D_2O$ exchanged, 6-NH). The spectrum is complicated by (presumably rotational) isomerism, giving doubling of some signals with roughly equal intensities; for clarity, only one set of resonances is quoted; (Found: $\underline{MH}^+$, 686. $C_{32}H_{39}N_5O_8S_2$ requires $\underline{M}$, 685).

(ii) <u>Benzyl 6β-[D-2-[(4-ethyl-2,3-dioxopiperazin-1-yl)-carbonylamino]-2-phenylacetamido]-6α-(methylthio)-penicillanate</u>

Benzyl 6β-[D-2-[[2-[N-ethyl-N-(methyloxalyl)amino]ethyl-amino]carbonylamino]-2-phenylacetamido]-6α-(methylthio)-penicillanate (60 mg, 0.088 mmol) was treated at room temperature with a 0.01 M solution of 1,8-diazabicyclo-[5.4.0]undec-7-ene in dichloromethane (1 ml). T.l.c. showed little starting material remaining after 0.5 h; after this time the solution was diluted with ethyl acetate (20 ml) and worked up for a neutral fraction as in Example 6. The crude product (54 mg) was chromatographed on silica gel 60; pooling and evaporation of appropriate fractions gave the title compound (24 mg, 42%), with identical spectral characteristics to those given in Example 3.

Example 8

Benzyl 6β-[D-2-[(4-ethyl-2,3-dioxopiperazin-1-yl)-carbonylamino]-2-phenylacetamido]-6α-(methylthio)-penicillanate

In addition to the use of 1,8-diazabicyclo [5.4.0]undec-7-ene in dichloromethane as in example 3, benzyl 6β-[D-2-[[2-[N-(4-nitrobenzyloxalyl) amino]ethylamino] carbonylamino]-2-phenylacetamido]-6α-(methylthio) penicillanate has been cyclised to the title compound under the following conditions:

a) Potassium carbonate in dimethylformamide; 0.25 h at room temperature.

b) Triethylamine in dichloromethane; 2 days at room temperature.

c) Triethylamine and 4-(dimethylamino)pyridine in dichloromethane; 2 days at room temperature.

d) 4-(Dimethylamino)pyridine in dichloromethane; 10 days at room temperature.

e) Potassium carbonate in acetone; 16 h at room temperature.

Example 9

Benzyl 6β-[D-2-[[2-[N-ethyl-N-(4-nitrobenzyloxalyl)amino]ethylamino]carbonylamino]-2-(3,4-diacetoxyphenyl)acetamido]-6α-(methylthio)penicillanate

Benzyl 6β-[D-2-(2,2,2-trichloroethoxycarbonylamino)-2-(3,4-diacetoxyphenyl)acetamido]-6α-(methylthio)penicillanate (0.777 g, 1 mmol) in tetrahydrofuran (15 ml) was treated with 1 M potassium dihydrogen phosphate and freshly acid washed zinc powder (1 g). The reaction mixture was stirred at pH 4 for 0.75 h before addition of more zinc (1 g). The reaction was continued at room temperature until complete by t.l.c. (total of 1.5 h). The reaction mixture was filtered and the filtrate evaporated to small volume and diluted with ethyl acetate. The solution was washed with brine, dried over $MgSO_4$ and then evaporated to leave benzyl 6β-[D-2-amino-2-(3,4-diacetoxyphenyl)acetamido]-6α-(methylthio)penicillanate. This, in dichloromethane (10 ml), was then added dropwise to a solution of 4-nitrobenzyl[N-(2-isocyanatoethyl)-N-ethylamino] oxalate (1 mmol) in dichloromethane ( 7 ml) at 0°C. The reaction solution was stirred at 0°C for 0.5 h followed by 1.5 h at room temperature, and then evaporated to dryness. Chromatography of the residue on silica gel 60 eluting with chloroform grading to 3% ethanol in chloroform afforded the title compound (0.231 g, 25%); $\nu_{max.}$ ($CH_2Cl_2$) 3380, 1778, 1745, 1655, 1525, 1350, 1210 $cm^{-1}$; n.m.r. showed signals due to two isomers, ($CDCl_3$) 1.01 (3H, s, 2-$CH_3$), 1.10 and 1.12 (3H, 2t, J 7Hz, 2 x $CH_2CH_3$), 1.21 (3H, s, 2-$CH_3$), 2.15 and 2.17 (3H, 2s, 2 x $SCH_3$), 2.23 and 2.26 (6H, 2s, 2 x $OCOCH_3$), 3.16 - 3.60 (6H, m, 3 x $NCH_2$), 4.33 (1H, s, 3-H), 5.13 (2H, s, $CH_2$Ph), 5.29 and 5.35 (2H, br.s and s, 2 x Ar $CH_2$), 5.46 and 5.47 (1H, 2s, 2 x 5-H), 5.74 (1H, m, $CH_2$NHCO), 5.92 and 6.00

(1H, 2d, J8Hz, 2 x CHNHCO), 6.72 (1H, m, CHNHCO), 7.00 and 7.02 (1H, 2d, J 8Hz, aromatic CH), 7.20 - 7.45 (7H, m, aromatic), 7.52 and 8.17, and 7.57 and 8.22 (4H, 2 x AA'BB', nitrophenyl), and 8.63 and 8.78 (1H, 2 br.s, 2 x 6β-NHCO).

Example 10

Benzyl 6β-[D-2-[(4-ethyl-2,3-dioxopiperazin-1-yl)carbonylamino]-2-(3,4-diacetoxyphenyl)acetamido]-6α-(methylthio)penicillanate

A solution of benzyl 6β-[D-2-[[2-[N-ethyl-N-(4-nitrobenzyloxalyl)amino]ethylamino]carbonylamino]-2-(3,4-diacetoxyphenyl)acetamido]-6α-(methylthio)penicillanate (0.092 g, 0.1 mmol) in dichloromethane (9 ml) was treated with a 0.1 M solution of 1,8-diazabicyclo[5.4.0]undec-7-ene in dichloromethane (1 ml, 0.1 mmol) and the reaction solution was stirred at room temperature for 0.25 h. It was then diluted with dichloromethane, washed with 0.5 M hydrochloric acid and brine, and then dried over magnesium sulphate. After evaporation to dryness, the crude product was chromatographed on silica gel 60 eluting with hexane/ethyl acetate 1:4 grading to ethyl acetate to afford the title compound (0.022 g, 29%); $\nu_{max.}$ ($CH_2Cl_2$) 3280, 1780, 1745, 1715, 1695, 1500, 1210, 1185 $cm^{-1}$; δ ($CDCl_3$) 0.95 (3H, s, 2-$CH_3$), 1.23 (6H, m, 2-$CH_3$ and $CH_2C\underline{H}_3$), 2.23 (3H, s, $SCH_3$), 2.24 and 2.26 (6H, 2s, 2 x $OCOCH_3$),3.55 (4H, m, piperazine $CH_2$ and $NC\underline{H}_2CH_3$), 3.90 - 4.09 (1H, m, piperazine CH), 4.09 - 4.22 (1H, m, piperazine CH), 4.32 (1H, s, 3-H), 5.13 and 5.17 (2H, ABq, *J* 12Hz, $C\underline{H}_2Ph$), 5.51 (1H, s, 5-H), 5.78 (1H, d, *J* 7Hz, NHC$\underline{H}$CO), 7.11 (1H, d, *J* 8Hz, aromatic H), 7.24 - 7.45 (7H, m, aromatics, 7.71 (1H, s, 6β-NHCO), and 10.18 (1H, d, *J* 7Hz, CHN$\underline{H}$CO) [Found: *m/z* (positive xenon F.A.B.) $MH^+$ 770. $C_{35}H_{39}N_5O_{11}S_2$ requires *M* 769].

Example 11

D-2-[[2-[N-Ethyl-N-(4-nitrobenzyloxalyl)amino]ethylamino]carbonylamino]-2-phenylacetic acid

D-Phenylglycine (0.302 g, 2 mmol) was suspended in hexamethyldisilazane (5 ml) containing chlorotrimethylsilane (0.5 ml) and heated at reflux under argon for 6 h. The resulting solution was evaporated to dryness, treated with toluene and re-evaporated. The residue was then dissolved in dichloromethane (10 ml), and a solution of 4-nitrobenzyl [N-(2-isocyanatoethyl)-N-ethylamino]oxalate (2 mmol) in dichloromethane (12 ml) was added. The reaction solution was stirred at room temperature for 16 h, before being evaporated to dryness. The residue was dissolved in ethyl acetate and 0.5 M hydrochloric acid was added. The two layers were separated and the organic phase was washed with 0.5 M hydrochloric acid before being extracted with dilute aqueous sodium hydrogen carbonate solution. The aqueous extracts were washed with ethyl acetate, saturated with sodium chloride and acidified to pH 1. The product was extracted into ethyl acetate, and the organic solution was washed with brine, dried and evaporated to leave the product as a white foam (0.358 g, 38%); $\nu_{max.}$ ($CH_2Cl_2$) 3400, 1730, 1660, 1630, 1525, 1350, 1210 $cm^{-1}$; n.m.r. showed signals from two isomers, $\delta$($CDCl_3$) 1.14 (3H, t, J 7Hz, $CH_2CH_3$), 3.00 - 3.70 (6H, m, 3 x $NCH_2$), 5.27 (2H, s, aryl-$CH_2$), 5.51 (1H, m, $\alpha$-CH rotamers), 5.82 (1H, br.s., exchangeable with $D_2O$, NH), 7.12 (1H, br.s., exchangeable with $D_2O$, NH), 7.20 - 7.50 (7H, m, aromatics) and 8.17 (2H, AA'BB', J 9 Hz, aromatics) [Found: m/z (positive xenon F.A.B.) $MH^+$ 473. $C_{22}H_{24}N_4O_8$ requires M 472].

Example 12

Benzyl 6β-[D-2-[[2-[N-ethyl-N-(4-nitrobenzyloxalyl)amino]ethylamino]carbonylamino]-2-phenylacetamido]-6α-(methylthio)penicillanate

A solution of D-2-[[2-[N-ethyl-N-(4-nitrobenzyloxalyl)amino]ethylamino]carbonylamino]-2-phenylacetic acid (0.139 g, 0.29 mmol) in dichloromethane (10 ml) was added slowly with stirring to a solution of benzyl 6β-amino-6α-(methylthio)penicillanate (0.102 g, 0.29 mmol) and N,N'-dicyclohexylcarbodiimide (0.066 g, 0.32 mmol) in dichloromethane (10 ml), and the reaction mixture was stirred at room temperature for 16 h. The solution was evaporated to dryness and the residue was chromatographed on silica gel 60 eluting with hexane/ethyl acetate 1:2 to afford the essentially pure title compound (0.024 g, 10%). T.l.c. and spectroscopic (i.r, n.m.r. and m.s.) data were identical to those of the material obtained by the method of example 1.

Example 13

D-2-[[2-[N-Ethyl-N-(4-nitrobenzyloxalyl)amino]ethylamino]carbonylamino]-2-(3,4-dihydroxyphenyl) acetic acid

D-3,4-Dihydroxyphenylglycine (0.915 g, 5 mmol) in hexamethyldisilazane (7 ml) containing chlorotrimethylsilane (0.7 ml) was refluxed under argon for 6 h. The resulting solution was evaporated to dryness and the residue was dissolved in dichloromethane (10 ml) and added to a solution of 4-nitrobenzyl [N-(2-isocyanatoethyl)-N-ethylamino]oxalate (5 mmol) in dichloromethane (25 ml). The reaction solution was stirred for 16 h at room temperature before being evaporated to dryness. The residue was dissolved in a mixture of ethyl acetate and 0.5 M hydrochloric acid and stirred for 5 minutes at room temperature. The mixture was adjusted to pH 8, the two phases were separated and the aqueous phase was washed with ethyl acetate. It was then saturated with sodium chloride, adjusted to pH 1.5 with hydrochloric acid, and the product was extracted into 50:50 ethyl acetate/tetrahydrofuran. The combined extracts were washed with brine, dried over magnesium sulphate, and evaporated to leave an oil (0.977 g). This was chromatographed on silica gel 60 eluting with ethyl acetate/iso-propanol/water 10:6:1 to afford the product as a pale brown foam (0.56 g, 22%); $\nu_{max.}$ (tetrahydrofuran) 3300, 1740, 1665, 1525, 1350 $cm^{-1}$; n.m.r. showed signals due to two isomers, $\delta[(CD_3)_2SO]$ 1.04 (3H, 2t, J 7Hz, $CH_2CH_3$), 3.02 - 3.42 (6H, m, 3 x $NCH_2$) 4.78 (1H, m, ArCH), 5.37 and 5.45 (2H, 2s, aryl-$CH_2$), 6.40 - 6.90 (4H, m, aromatics and NH), 7.67, 7.70, 8.24, and 8.27 (4H, AA'BB', J 10.5 Hz, aromatics) and 8.50 - 9.30 (2H, br.s, NH and $CO_2H$). [Found: m/z (positive xenon F.A.B.) $MH^+$ 505. $C_{22}H_{24}N_4O_{10}$ requires M 504].

Example 14

D-2-[[2-[N-Ethyl-N-(4-nitrobenzyloxalyl)amino] ethylamino]carbonylamino]-2-(3,4-diacetoxyphenyl) acetic acid

The 3,4-dihydroxy acid of example 13 (0.252 g, 0.5 mmol) in tetrahydrofuran (2 ml) was treated with water (10 ml) and the pH was adjusted to 7.5 with dilute aqueous sodium hydrogen carbonate solution. Acetic anhydride (0.112 g, 1.1 mmol) was then added, and the pH re-adjusted to 7.5. The reaction was allowed to proceed for 1.5 h at room temperature. T.l.c. indicated incomplete reaction so more acetic anhydride (0.112 g, 1.1 mmol) was added and reaction was continued for a further hour. The solution was then washed with ethyl acetate, saturated with sodium chloride, and the pH adjusted to 1.5 with 1 M hydrochloric acid. The product was extracted into 50:50 tetrahydrofuran/ethyl acetate, the extracts were washed with brine, dried over magnesium sulphate and then evaporated to dryness to afford the product (0.048 g, 16%); $\nu_{max.}$($CH_2Cl_2$) 3390, 1765, 1730, 1660, 1530, 1370, 1350 $cm^{-1}$; $\delta$($CD_3OD$) 1.13 (3H, t, J 7 Hz, $NCH_2CH_3$), 2.22 (6H, s, 2 x $OCOCH_3$), 3.10 - 3.60 (6H, m, 3 x $NCH_2$), 5.10 - 5.50 (3H, m, aryl-$CH_2$ and α-CH rotamers), 6.60 - 7.35 (3H, m, aromatics), 7.59, 7.64, 8.21, and 8.25 (4H, 2AA'BB', J 9Hz, aromatics, rotamers).

Example 15

Benzyl 6β-[D-2-[[2-[N-ethyl-N-(4-nitrobenzyloxalyl)amino]ethylamino]carbonylamino]-2-(3,4-diacetoxyphenyl)acetamido]-6α-(methylthio)penicillanate

A solution of D-2-[[2-[N-ethyl-N-(4-nitrobenzyloxalyl)amino]ethylamino]carbonylamino]-2-(3,4-diacetoxyphenyl)acetic acid (0.048 g, 0.08 mmol) in dichloromethane (5 ml) was added dropwise over 1.5 h to a stirred solution of benzyl 6β-amino-6α-(methylthio)penicillanate (0.028 g, 0.08 mmol) and N,N'-dicyclohexylcarbodiimide (0.018 g, 0.088 mmol) in dichloromethane (3 ml). The reaction mixture was stirred overnight at room temperature before being evaporated to dryness and chromatographed on silica gel 60 eluting with chloroform grading to 2% ethanol/chloroform to afford the slightly impure product (0.009 g, 12%). T.l.c. and spectrosccopic (i.r., n.m.r. and m.s.) data were identical with those obtained from the compound of example 9.

Example 16

Benzyl 6β-[D-2-[[2-[N-ethyl-N-(2,2,2-trichloroethoxycarbonyl)amino]ethylamino]carbonylamino]-2-(3,4-diacetoxyphenyl)acetamido]-6α-(methylthio)penicillanate

Benzyl 6β-[D-2-(2,2,2-trichloroethoxycarbonylamino)-2-(3,4-diacetoxyphenyl)acetamido]-6α-(methylthio) penicillanate (1.55 g, 2 mmol) was dissolved in tetrahydrofuran (30 ml) and 1M potassium dihydrogen phosphate (6 ml) was added. The mixture was stirred vigorously, freshly acid-washed zinc (3 g) was added and the pH maintained in the region 3.0 - 4.0 by addition of hydrochloric acid, the reaction being monitored by t.l.c. After 1 h a further portion of zinc (3 g) was added; the reaction appeared complete by t.l.c. in a further 1 h. The insoluble material was filtered off and washed well with water and ethyl acetate; the organic phase was separated and washed further with water (2 x 50 ml) and brine, then dried over anhydrous magnesium sulphate and evaporated to dryness. The residue was redissolved in anhydrous dichloromethane (10 ml) and stirred at 0°C with exclusion of moisture. To this was added over 2 min. a solution of 2-[N-ethyl-N-(2,2,2-trichloroethoxycarbonyl)amino]ethyl isocyanate in the same solvent (10 ml) prepared from the corresponding amine hydrochloride (2 mmol) as described in Example 7. The resulting solution was allowed to regain room temperature, then stirred for a further 1 h and concentrated to near dryness. Workup for a neutral fraction as described in Example 6 was followed by chromatography on silica gel (30 g), eluting with ethyl acetate: hexane, 1:1. Appropriate fractions were combined and evaporated to give the title compound (0.92 g, 52%); $R_F$ 0.38 in 5% methanol-chloroform; $\nu_{max.}$ (K Br) 1777, 1745, 1714, 1641, 1544, 1499 $cm^{-1}$;

$[(CD_3)_2CO]$ 1.15 (6H, s + t, $CH_3CH_2N$ + one $2-CH_3$), 1.25 (3H, s, $2-CH_3$), 2.24, 2.26 (6H, 2s, 2 x $CH_3CO$), 2.32 (3H, s, $CH_3S$), 3.20 - 3.50 (6H, m, 3 x $CH_2N$), 4.41 (1H, s, 3-H), 4.79, 4.83 (2H, ABq, $OCH_2CCl_3$), 5.21 (2H, s, $PhCH_2O$), 5.41 (1 H, s, 5-H), 5.65 (1H, d, s on $D_2O$ exchange, CHNH), 6.00 (1 H, br m, $D_2O$ exchanged, NH), 6.47 (1H, approx. t, $D_2O$ exchanged, NH), 7.10 - 7.50 (8H, m, Ar H), 8.84 (1 H, br s, $D_2O$ exchanged, 6-NH); (Found: $MH^+$, 890 $C_{36}H_{42}Cl_3N_5O_{11}S_2$ requires M 889).

Example 17



Benzyl 6β-[D-2-[(4-ethyl-2,3-dioxopiperazin-1-yl)carbonylamino]-2-(3,4-diacetoxyphenyl)acetamido]-6α-(methylthio)penicillanate

(i) Benzyl 6β-[D-2-[[2-[N-ethyl-N-(methyloxalyl)amino]ethylamino]carbonylamino]-2-(3,4-diacetoxyphenyl)acetamido]-6α-(methylthio)penicillanate

Benzyl 6β-[D-2-[[2-[N-ethyl-N-(2,2,2-trichloroethoxycarbonyl)amino]ethylamino]carbonylamino]-2-(3,4-diacetoxyphenyl)acetamido]-6α-(methylthio)penicillanate (450 mg, 0.5 mmol) was dissolved in tetrahydrofuran (20 ml) and 1 M potassium dihydrogen phosphate (4 ml) was added. Freshly acid-washed zinc (1 g) was added to the vigorously stirred mixture and the pH was maintained at about 3.5 - 4.0 by addition of hydrochloric acid. Further portions of zinc (2 x 1 g) were added after 1.5 h and 2 h, and after 2.5 h the reaction appeared complete by t.l.c. The mixture was filtered and the solids were washed with water and tetrahydrofuran, then the aqueous phase was saturated with sodium chloride and the organic phase was separated. The aqueous phase was extracted once more with tetrahydrofuran, then the total organic extract was dried over magnesium sulphate and rigorously evaporated to dryness. Without further purification, this material was redissolved in anhydrous dichloromethane (10 ml), cooled to 0°C and stirred under argon with triethylamine (0.14 ml, 1 mmol). Methyl oxalyl chloride (0.06 ml, 0.65 mmol) was added and the reaction was monitored by t.l.c. After 0.25 h the reaction mixture was worked up for a neutral product as described in Example 6. Chromatography on silica gel (15 g), eluting firstly with ethyl acetate:hexane 2:1, then with ethyl acetate, gave on evaporation of appropriate fractions the title product (235 mg, 59%); $R_F$ 0.53 in 10% methanol-

chloroform; $\nu_{max.}$ (KBr) 1777, 1743, 1653, 1541, 1500 $cm^{-1}$; $\delta$[$(CD_3)_2CO$] 1.15, 1.26 [6H, 2s, $(CH_3)_2C$], 1.14 (3H, m, $\underline{CH_3}CH_2N$ rotamers), 2.24, 2.26 (6H, 2s, 2 x $CH_3CO$), 2.32 (3H, s, $CH_3S$), 3.20 - 3.50 (6H, m, 3 x $CH_2N$), 3.75 and 3.83 (3H, 2s, $CH_3O_2C$ rotamers), 4.41 (1H, s, 3-H), 5.21 (2H, s, $Ph\underline{CH_2}O$), 5.65 (1H, d, $C\underline{H}NH$), 6.00 (1H, approx. t, $D_2O$ exchanged, $N\underline{H}CH_2$), 6.50 (1H, approx. t, $D_2O$ exchanged, $N\underline{H}CH$ rotamers), 7.10 - 7.50 (8H, m, Ar H), 8.80 (1H, brs, $D_2O$ exchanged, 6-NH); (Found; $\underline{MH}^+$, 802. $C_{36}H_{43}N_5O_{12}S_2$ requires $\underline{M}$ 801).

(ii) Benzyl 6β-[D-2-[(4-ethyl-2,3-dioxopiperazin-1-yl)carbonylamino]-2-(3,4-diacetoxyphenyl)acetamido]-6α-(methylthio)penicillanate

Benzyl 6β-[D-2-[[2-[N-ethyl-N-(methyloxalyl)amino]ethylamino]carbonylamino]-2-(3,4-diacetoxyphenyl)acetamido]-6α-(methylthio)penicillanate (46 mg, 0.057 mmol) was dissolved in dry dichloromethane (1 ml) and treated at ambient temperature with a 0.1 M solution of 1,8-diazabicyclo[5.4.0]undec-7-ene in the same solvent (0.6 ml). After 10 min. little starting material was visible on t.l.c.; the solution was diluted with ethyl acetate (20 ml) and worked up for a neutral product as in Example 6. Chromatography on silica gel (2.5 g), eluting firstly with ethyl acetate: hexane 2:1, then ethyl acetate, gave on evaporation of appropriate fractions the title compound (23 mg, 52%), with spectral characteristics identical to those given in Example 10.

Example 18

Benzyl 6β-[D-2-[[2-[N-ethyl-N-(methyloxalyl) amino] ethylamino]carbonylamino]-2-(3,4-diacetoxyphenyl) acetamido]-6α-formamidopenicillanate

Benzyl 6β-[D-2-[[2-[N-ethyl-N-(methyloxalyl)amino] ethylamino]carbonylamino]-2-(3,4-diacetoxyphenyl) acetamido]-6α-(methylthio)penicillanate (160 mg, 0.20 mmol) in anhydrous dimethylformamide (3 ml) was stirred at -40°C under argon. Mercury (II) acetate (64 mg, 0.20 mmol) was added, followed by a solution of ammonia (3.5 mg, 0.20 mmol) in dimethylformamide (0.16 ml). The mixture was allowed to regain ambient temperature; after 0.75 h no starting material was visible on t.l.c. After 1 h the mixture was partitioned between ethyl acetate (20 ml) and water (20 ml); the organic phase was separated and washed further with water (4 x 20 ml) and brine, then dried over magnesium sulphate and evaporated to dryness. Without further purification this intermediate was redissolved in anhydrous dichloromethane (3 ml) and stirred at 0°C; anhydrous pyridine (0.2 ml, 2.48 mmol) was added, followed by acetic formic anhydride (0.10 ml, 1.24 mmol). The solution was allowed to regain ambient temperature, then poured into ethyl acetate (20 ml) and washed sequentially with 0.5 M hydrochloric acid (2 x 10 ml), saturated aqueous sodium hydrogen carbonate (2 x 10 ml), water and brine. The final organic phase was dried over anhydrous magnesium sulphate, then evaporated to a crude product (150 mg) which was purified by chromatography on silica gel, eluting firstly with 2% methanol in chloroform, then 3%, and finally 4%. Product-rich fractions were combined and evaporated to give the title compound (102 mg, 64%); $R_F$0.41 in 10% methanol-chloroform; $\nu_{max.}$ (KBr) 1772, 1743, 1653 br, 1544, 1499 $cm^{-1}$; $\delta[(CD_3)_2CO]$ 1.05, 1.23 [6H, 2s, $(CH_3)_2C$],

1.10, 1.14 (3H, 2t, $C\underline{H}_3CH_2N$, rotamers), 2.24, 2.26 (6H, 2s, 2 x $CH_3CO$), 3.20 - 3.50 (6H, m, 3 x $CH_2N$), 3.74, 3.83 (3H, 2s, $CH_3O_2C$ rotamers), 4.40 (1H, s, 3-H), 5.19 (2H, s, $PhC\underline{H}_2O$), 5.58 (1H, s, 5-H), 5.61 (1H, approx d, s on $D_2O$ exchange, C<u>H</u>NH), 6.01 (1H, br s, $D_2O$ exchanged, NH), 6.40 - 6.60 (1H, m, $D_2O$ exchanged, N<u>H</u>CH), 7.10 - 7.50 (8H, m, ArH), 8.10 - 8.20 (2H, m, approx. s on $D_2O$ exchange, N<u>H</u>C<u>H</u>O), 8.79 (1H, br s, $D_2O$ exchanged, 6-NH), (Found: <u>M-H</u>, 797. $C_{36}H_{42}N_6O_{13}S$ requires <u>M</u>, 798).

Example 19

Sodium 6β-[D-2-[[2-[N-ethyl-N-(methyloxalyl)amino]ethylamino]carbonylamino]-2-(3,4-diacetoxyphenyl)acetamido]-6α-formamidopenicillanate

Benzyl 6β-[D-2-[[2-[N-ethyl-N-(methyloxalyl)amino]ethylamino]carbonylamino]-2-(3,4-diacetoxyphenyl)acetamido]-6α-formamidopenicillanate (92 mg, 0.12 mmol) was dissolved in tetrahydrofuran (10 ml). 10% Palladium on charcoal (100 mg) was added and the mixture was hydrogenated under ambient conditions; after 1.75 h no starting material was visible by t.l.c. The catalyst was filtered and well washed with tetrahydrofuran, and the filtrate was evaporated to dryness. The residue was redissolved in acetone (2 ml) and treated with a solution of sodium 2-ethylhexanoate in 4-methylpentan-2-one (1 equivalent). Precipitation was completed by diluting to 15 ml with ether; the white precipitate was filtered, washed with acetone: ether 1:1 and ether and dried to give the title penicillin (68 mg, 81%); $R_F$0.26 in n-butanol:acetic acid:water, 4:1:1; $\nu_{max.}$ (KBr) 1771, 1653 br, 1610 sh, 1553, 1500 $cm^{-1}$; $\delta(D_2O)$ 0.95, 1.29 [6H, 2s, $(CH_3)_2C$], 1.14, 1.15 (3H, 2t, $CH_3CH_2N$ rotamers), 2.33, 2.34 (6H, 2s, 2 x $CH_3CO$), 3.15 - 3.65 (6H, m, 3 x $CH_2N$), 3.78, 3.89 (3H, 2s, $CH_3O_2C$ rotamers), 4.18 (1H, s, 3-H), 5.34 (1H, s, CHNH), 5.59 (1H, s, 5-H), 7.25 - 7.55 (3H, m, ArH), 8.13 (1H, s, NHCHO); (Found: $MH^+$, 731. $C_{29}H_{35}N_6NaO_{13}S$ requires M, 730).

Example 20

t-Butyl 7β-[D-2-[[2-[N-ethyl-N-(4-nitrobenzyloxalyl)amino]ethylamino]carbonylamino]-2-phenylacetamido]-7α-formamidocephalosporanate

A solution of t-Butyl 7β-(D-2-azido-2-phenylacetamido)-7α-formamidocephalosporanate (0.267 g, 0.5 mmol) and ammonium chloride (0.178 g, 3.3 mmol) in methanol (8 ml) and tetrahydrofuran (4 ml) was treated with freshly acid washed zinc powder (0.1 g), and the reaction mixture was stirred at room temperature for 0.5 h. More zinc (0.2 g) was then added and stirring at room temperature continued for a further 5 minutes. The reaction mixture was filtered and the filtrate was evaporated to dryness. The residue was dissolved in dichloromethane, dried over magnesium sulphate, and then the solution was evaporated to dryness to leave the crude t-butyl 7β-(D-2-amino-2-phenylacetamido)-7α-formamidocephalosporanate. This was dissolved in dichloromethane (5 ml) and added dropwise to a solution of 4-nitrobenzyl[N-(2-isocyanatoethyl)-N-ethylamino]oxalate (0.5 mmol) in dichloromethane (10 ml) at 0°C. The reaction solution was stirred at 0°C for 0.5 h and then for 1 h at room temperature before being washed with 0.5 M hydrochloric acid and brine. The solution was dried over magnesium sulphate, evaporated to dryness and the residue chromatographed on silica gel 60 eluting with chloroform grading to 5% ethanol/chloroform to afford the desired product (0.075 g, 18%); $\nu_{max.}(CH_2Cl_2)$ 3375, 1790, 1745, 1690, 1655, 1530, 1350, 1210 $cm^{-1}$; n.m.r. showed signals from two isomers, δ$(CDCl_3)$ 1.09 (3H,2t, $CH_2C\underline{H}_3$), 1.50 (9H, s, t-butyl), 2.06 (3H, s, $OCOCH_3$), 2.99 and 3.01 (1H, half of ABq, J 18Hz, 2-H), 3.15 - 3.70 (7H, m, 3 x $CH_2N$ and 2-H), 4.78 and 4.95 (2H, ABq, J 13Hz, $C\underline{H}_2OAc$), 5.13 and 5.19 (2H, ABq, J 12Hz, $C\underline{H}_2Ar$),

5.28 and 5.30 (1H, 2s, 6-H), 5.79 (1H, m, PhCHNH isomers), 5.99 (1H, m, $CH_2NHCO$ isomers), 7.70 (1H, m, PhCHNH isomers), 7.10 - 7.60 (7H, m, aromatics), 7.98 and 8.02 (1H, 2s, CHO isomers), 8.18 (2H, half of AA'BB', *J* 9Hz, aromatics), 8.39 (1H, br.s, NHCHO) and 8.67 (1H, m, 7β-NH isomers). [Found: *m/z* (positive xenon F.A.B.) *M*$H^+$ 826. $C_{37}H_{43}N_7O_{13}S$ requires *M* 825].

Example 21

t-Butyl 7β-[D-2-[(4-ethyl-2,3-dioxopiperazin-1-yl)carbonylamino]-2-phenylacetamido]-7α-formamidocephalosporanate

(i) t-Butyl 7β-[D-2-[(4-ethyl-2,3-dioxopiperazin-1-yl)carbonylamino]-2-phenylacetamido]-7α-formamido-3-(acetoxymethyl)ceph-2-em-4-carboxylate

A solution of t-butyl 7β-[D-2-[[2-[N-ethyl-N-(4-nitrobenzyloxalyl)amino]ethylamino]carbonylamino]-2-phenylacetamido]-7α-formamidocephalosporanate (0.052g, 0.063 mmol) in dichloromethane (12 ml) was treated with a 1M solution of 1,8-diazabicyclo[5.4.0]-undec-7-ene in dichloromethane (0.630 ml, 0.063 mmol) and the reaction solution was stirred at room temperature for 0.25 h. It was then washed with 0.5 M hydrochloric acid and brine, and then dried over magnesium sulphate before being evaporated to dryness. The crude product was chromatographed on silica gel 60 eluting with 5% ethanol/chloroform to afford the title compound (0.018 g, 42%); $\nu_{max.}(CH_2Cl_2)$ 3280, 1790, 1740 sh, 1720, 1690, 1495, 1360 $cm^{-1}$; n.m.r. showed mainly the title compound plus ≃10% Δ3 isomer, $\delta(CDCl_3)$ 1.23 (3H, t, J 7Hz, $CH_2CH_3$), 1.50 (9H, s, t-butyl), 2.05 (3H, s, $OCOCH_3$), 3.54 (3H, m, $CH_2CH_3$ and piperazine CH), 3.72 (1H, m, piperazine CH), 3.93 (1H, m, piperazine CH), 4.13 (1H, m, piperazine CH), 4.49 and 4.60 (2H, ABq, J 13Hz, $CH_2OAc$), 4.83 (1H, s, 4-H), 5.40 (1H, s, 6-H), 5.54 (1H, d, J 7Hz, NHCHO), 6.02 (1H, s, 2-H), 7.18 - 7.53 (5H, m, aromatics), 7.90 (1H, s, NHCO), 8.12 (1H, s, CHO), 8.32 (1H, br.s, NHCO), and 9.98 (1H, d, J 7Hz, NHCHO), [Found: m/z (positive xenon F.A.B.) $MH^+$, 673. $C_{30}H_{36}N_6O_{10}S$ requires M, 672]

(ii) t-Butyl 7β-[D-2-[(4-ethyl-2,3-dioxopiperazin-1-yl)carbonylamino]-2-phenylacetamido]-7α-formamidocephalosporanate-1-oxide

A solution of t-butyl 7β-[D-2-[(4-ethyl-2,3-dioxopiperazin-1-yl)carbonylamino]-2-phenylacetamido]-7α-formamido-3-(acetoxymethyl)ceph-2-em-4-carboxylate (0.017 g, 0.025 mmol) in dichloromethane (2 ml) was treated with a solution of 3-chloroperoxybenzoic acid (0.0047 g, 0.0275 mmol) in dichloromethane (1 ml). The reaction solution was stirred at room temperature for 0.75 h, and then more 3-chloroperoxybenzoic acid (0.0023 g) in dichloromethane (0.5 ml) was added. After stirring for a further 0.25 h the solution was diluted with dichloromethane and washed with dilute sodium hydrogen carbonate solution and brine. It was dried over magnesium sulphate, filtered and evaporated to leave the crude product, which was chromatographed on silica gel 60 eluting with ethyl acetate grading to 5% ethanol/ethyl acetate to afford the two sulphoxide isomers. The less polar isomer (0.008 g, 47%) had $\nu_{max.}$ ($CH_2Cl_2$) 3260, 1800, 1740 sh, 1720, 1690, 1680 sh 1500, 1220, 1040 $cm^{-1}$; $\delta[(CD_3)_2CO]$ 1.18 (3H, t, $\underline{J}$ 7Hz, $CH_2C\underline{H}_3$), 1.54 (9H, s, t-butyl), 2.00 (s, $OCOCH_3$, obscured by $CD_3COCHD_2$), 3.52 (2H, q, $\underline{J}$ 7Hz, $C\underline{H}_2CH_3$), 3.57 - 3.79 (4H, m, piperazine $CH_2$ and 2-$H_2$), 4.05 (2H, m, piperazine $CH_2$), 4.58 and 4.67 (1H, 2 x half of ABq, $\underline{J}$ 13Hz, $C\underline{H}OCOCH_3$ rotamers), 4.93 and 5.03 (1H, 2s, 6-H rotamers), 5.13 and 5.25 (1H, 2 x half of ABq, $\underline{J}$ 13Hz, $C\underline{H}OCOCH_3$ rotamers), 5.70 (1H, d, $\underline{J}$ 7Hz, PhC$\underline{H}$NH-), 7.34 (3H, m, aromatics), 7.53 (2H, m, aromatics), 8.17 (0.75H, s,NHC$\underline{H}$O rotamer), 8.42 (1H, s, 7β-NH), 8.54 (0.25H, d, $\underline{J}$ 11Hz, NHC$\underline{H}$O rotamer), 8.93 and 9.25 (1H, 2s, N$\underline{H}$CHO rotamers) and 9.96 (1H, d, $\underline{J}$ 7Hz, PhCHN$\underline{H}$-). [Found: m/z (positive xenon F.A.B. with $NH_4SCN$) $\underline{M}NH_4^+$, 706. $C_{30}H_{36}N_6O_{11}S$ requires $\underline{M}$, 688].

The more polar isomer (0.007g, 41%) had $\nu_{max.}(CH_2Cl_2)$ 3260; 1800, 1735 sh, 1715, 1690, 1500, 1220, 1040 $cm^{-1}$; $\delta$ [$(CD_3)_3CO$] 1.17 (3H, t, J 7Hz, $CH_2C\underline{H}_3$), 1.52 (9H, s, t-butyl), 2.07 (s, $OCOCH_3$, obscured by $CD_3COCHD_2$), 3.51 (2H,,q, J 7Hz, $C\underline{H}_2CH_3$), 3.57 and 3.83 (2H, ABq, J 16Hz, $2-H_2$), 3.72 (2H, m, piperazine $CH_2$), 4.08 (2H, m, piperazine $CH_2$), 4.75 (1H, s, 6-H), 4.78 and 5.21 (2H, ABq, J 13Hz, $C\underline{H}_2OCOCH_3$), 5.73 (1H, d, J 7Hz, PhC$\underline{H}$NH), 7.40 (3H, m, aromatics), 7.54 (2H, m, aromatics), 8.27 (1H, s, NHC$\underline{H}$O), 8.85 (1H, s, NHCO), 9.00 (1H, s, NHCO), and 9.96 (1H, d, J 7Hz, PhCHN$\underline{H}$). [Found: m/z (positive xenon F.A.B. with $NH_4SCN$) $\underline{M}NH_4^+$, 706. $C_{30}H_{36}N_6O_{11}S$ requires M, 688].

(iii) t-Butyl 7β-[D-2-[(4-ethyl-2,3-dioxopiperazin-1-yl)carbonylamino]-2-phenylacetamido]-7α-formamidocephalosporanate

The corresponding sulphoxide (0.100g, 0.145 mmol) in dichloromethane (10 ml) was cooled to 0°C and then treated with a solution of phosphorous trichloride (0.059g, 0.434 mmol) in dichloromethane (1 ml). The reaction solution was stirred at 0°C for 1.25 h and then diluted with ethyl acetate. The solution was washed with dilute sodium hydrogen carbonate and brine, before being dried over magnesium sulphate and evaporated to dryness. The crude product was chromatographed on silica gel 60 eluting with ethyl acetate grading to 5% ethanol/ethyl acetate to afford the title compound (0.035g, 36% from the more polar sulphoxide isomer; 0.023g, 23% from the less polar sulphoxide isomer); $\nu_{max.}$ $(CH_2Cl_2)$ 3270, 1790, 1740 sh, 1720, 1690, 1495, 1220 $cm^{-1}$; $\delta$[$(CD_3)_2CO$] 1.17 (3H, t, J 7Hz, $CH_2C\underline{H}_3$), 1.51 (9H, s, t-butyl), 2.01 (3H, s, $OCOCH_3$), 3.16 and 3.49 (2H, ABq, J 17Hz, $2-H_2$), 3.51 (2H, q, J 7Hz, $C\underline{H}_2CH_3$), 3.70 (2H, m, piperazine $CH_2$), 4.03 (2H, m, piperazine $CH_2$), 4.70 and 4.98 (2H, ABq, J 13Hz, $C\underline{H}_2OCOCH_3$), 5.18 (1H, s, 6-H), 5.73 (1H, d, J 7 Hz, PhC$\underline{H}$NH), 7.35 (3H, m, aromatics), 7.57 (2H, m,

aromatics), 8.20 (1H, s, NHC*H*O), 8.40 (1H, s, NHCO), 8.71 (1H, s, NHCO) and 9.99 (1H, d, *J* 7Hz, PhCHN*H*). [Found m/z (positive xenon F.A.B with $NH_4SCN$) *M*$NH_4^+$, 690. $C_{30}H_{36}N_6O_{10}S$ requires *M*, 672].

Example 22

t-Butyl 7β-[D-2-[(4-ethyl-2,3-dioxopiperazin-1-yl)carbonylamino]-2-phenylacetamido]-7α-formamido-3-(acetoxymethyl)ceph-2-em-4-carboxylate

In addition to the use of 1,8-diazabicyclo[5.4.0]undec-7-ene in dichloromethane as in example 21, t-butyl 7β-[D-2-[[2-[N-ethyl-N-(4-nitrobenzyloxalyl)amino]-ethylamino]carbonylamino]-2-phenylacetamido]-7α-formamidocephalosporanate has been cyclised to the title compound under the following conditions:

a) Triethylamine in dichloromethane; 1 day at room temperature

b) Pyridine plus 4-(dimethylamino)pyridine in dichloromethane; 10 days at room temperature.

c) N-Methylmorpholine in dichloromethane; 17 days at room temperature.

d) N,N-di-isopropylethylamine in dichloromethane; 6 days at room temperature.

e) Morpholine in dichloromethane; 5 days at room temperature.

Example 23

(a) D-2-[[2-[N-Ethyl-N-(ethyloxalyl)amino]ethylamino]-carbonylamino]-2-(3,4-dihydroxy)phenyl acetic acid

A solution of 2-[N-ethyl-N-(ethyloxalyl)amino]ethyl ammonium chloride (6.4g, 28.5 mmole) in M.D.C. (200 ml) was cooled to -10°. To this solution was added a solution of phosgene in toluene (39 ml of a 12.5% w/w solution) followed by a solution of triethylamine (8.55 g, 85 mmol) in M.D.C. (25 ml) at the same temperature. Stirring was continued for 20 mins during which time precipitation occurred. This mixture was cooled to -15° and reserved.

3,4-dihydroxyphenyl glycine (5.2 g, 28.5 mmol) was suspended in hexamethyldisilazane (80 ml) and trimethylchlorosilane (1 ml) and refluxed for 30 minutes. The solution was cooled and stirred in vacuo and redissolved in M.D.C. (20 ml). This solution was added to the mixture prepared earlier over 15 minutes and the total then stirred to room temperature. The mixture was stripped to low volume and partitioned between ethyl acetate and 2M hydrochloric acid (100/50 ml) and the aqueous rejected. The solution was washed with water (3 x 50 ml) and extracted with sodium bicarbonate solution. The aqueous was washed with ethyl acetate and then covered with 1:1 THF/ethyl acetate (50 ml/50 ml), saturated with sodium chloride and the pH adjusted to 1 with conc. hydrochloric acid. The organic layer was washed with brine and stripped in vacuo to afford the title compound as a white foam (6.0 g, 15.1 mmol, 53%).

(b) D-2-[[2-[N-Ethyl-N-(ethyloxalyl)amino]ethylamino] carbonylamino]-2-(3,4-diacetoxy)phenyl acetic acid.

The corresponding 3,4-dihydroxy acid (5.5 g, 13.8 mmol) in 1:1 THF/water (140 ml) was brought to pH 7 with aqueous sodium carbonate solution. Acetic anhydride (20 ml) was added and the pH left at 6.5 - 7.0 with sodium carbamate solution. When the pH steadied (ca 20 min) a further 20 ml acetic anhydride was added the process repeated. The final solution (at pH 7.5) was stripped to remove THF and washed with ethyl acetate. The aqueous phase was covered with ethyl acetate (100 ml) and the pH adjusted to 1 with conc. hydrochloric acid. The organic phase was washed with brine (50 ml), dried, and stripped to an oil. This oil was triturated with hexane to remove acetic acid and the resultant oil dried in vacuo to afford an off white foam 5.5 g, 11.4 mmol, 83%.

Example 24

D-2-[[2-[N-Ethyl-N-(methyloxalyl)amino]ethylamino] carbonylamino]-2-(3,4-dihydroxy)phenyl acetic acid

This compound was prepared from 2-[N-ethyl-N-(methyloxalyl) amino]ethyl ammonium chloride by the method described in Example 23.

Example 25

D-2-[[2-[N-Ethyl- N-(t-butyloxalyl)amino]-2-(3,4-dihydroxy) phenyl acetic acid

This compound was prepared from 2-[N-ethyl-N-(t-butyloxalyl) amino]ethyl ammonium chloride by the method described in Example 23.

A

Claims

1. A compound having the partial structure (II);

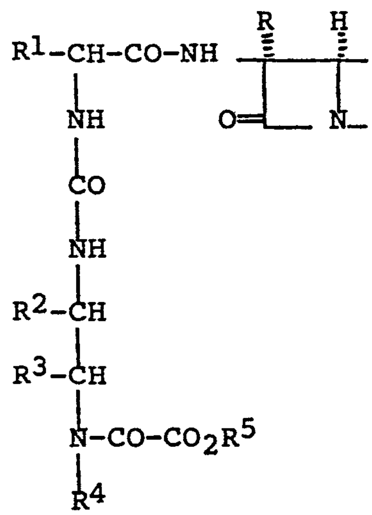

(II)

wherein: R represents $C_{1-6}$ alkylthio, arylthio, methoxy, hydroxymethyl, amino or formamido;

$R^1$ represents hydrocarbon or heterocyclyl;

$R^2$ and $R^3$ are the same or different and each represents hydrogen, optionally substituted $C_{1-6}$ alkyl, halogen, aryl, amino, hydroxy or $C_{1-6}$ alkoxy or $R^2$ and $R^3$ form the residue of a 5- or 6-membered carbocyclic or heterocyclic ring;

$R^4$ represents hydrogen, or hydrocarbon;

and $R^5$ represents hydrogen or a group which allows the $-CO_2R^5$ group to effect N-acylation.

2. A compound of formula (IV):

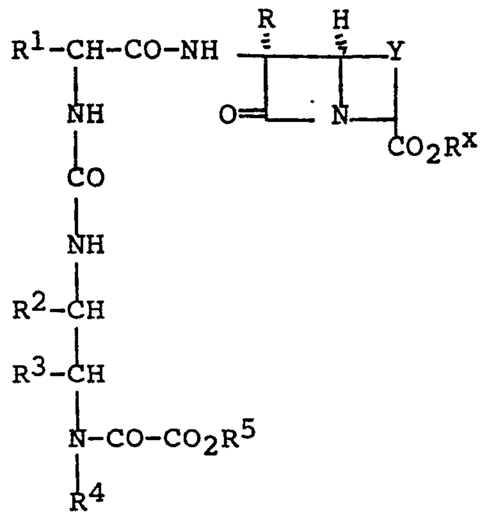

(IV)

wherein: R represents $C_{1-6}$ alkylthio, arylthio, methoxy, hydroxymethyl, amino or formamido; Y represents

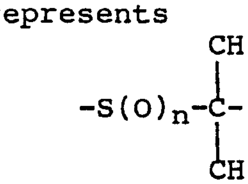 , $-S(O)_n-CH_2-$ ,

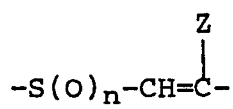 , 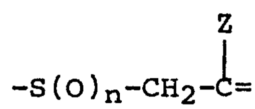 ,

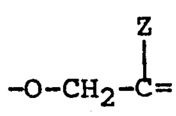 , 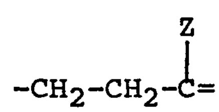 ,

wherein n is zero, 1 or 2 and Z represents hydrogen,halogen, or an organic group such as $C_{1-4}$ alkoxy, $-CH_2Q$ or $-CH=CH-Q$ wherein Q represents

hydrogen, halogen, hydroxy, mercapto, cyano, carboxy, carboxylic ester, carbamoyloxy, $C_{1-4}$ alkyloxy, acyloxy, aryl, a heterocyclyl group bonded via carbon, a heterocyclylthio group or a nitrogen containing heterocyclic group bonded via nitrogen;
$R^1$ represents hydrocarbon or heterocyclyl;

$R^2$ and $R_3$ are the same or different and each represents hydrogen, optionally substituted $C_{1-6}$ alkyl, halogen, aryl, amino, hydroxy or $C_{1-6}$ alkoxy or $R^2$ and $R^3$ form the residue of a 5- or 6-membered carbocyclic or heterocyclic ring;

$R^4$ represents hydrogen, or hydrocarbon;

$R^x$ is hydrogen or a carboxyl blocking group;

and $R^5$ represents hydrogen or a group which allows the $-CO_2R^5$ group to effect N-acylation.

3. A compound as claimed in claim 2 in which Y is $-S-C(CH_3)_2-$ or $-S-CH_2-CZ=$ where Z is as defined in respect of formula (IV).

4. A compound as claimed in claim 2 or claim 3 in which R is formamido and $R^1$ is 3,4-dihydroxyphenyl, 3,4-diacetoxyphenyl or 3,4-dibenzyloxycarbonyloxyphenyl.

5. A process for the preparation of a compound as claimed in claim 1 which comprises reacting a compound having the partial structure (V):

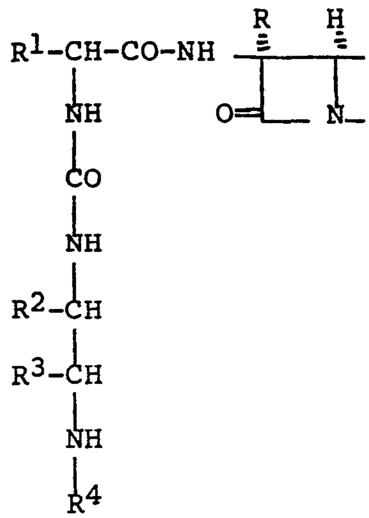

(V)

wherein R, $R_1$, $R^2$, $R^3$ and $R^4$ are as defined with respect to formula (II); with a reactive N-acylating derivative of an acid of formula (VI):

$$R^5O_2C.CO_2H \qquad (VI)$$

wherein $R^5$ is as defined with respect to formula (II).

6. A process for the preparation of a compound as claimed in claim 2 which comprises reacting a compound of formula (Va):

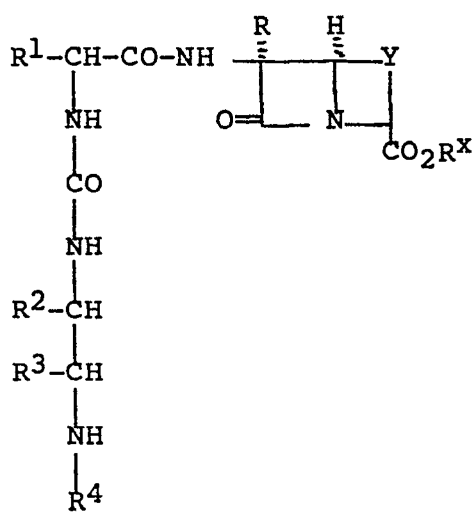

(Va)

wherein R, $R^1$, $R^2$, $R^3$, $R^4$, $R^x$ and Y are as defined with respect to formula (IV); with a reactive N-acylating derivative of an acid of formula (VI):

$$R^5O_2C.CO_2H \qquad (VI)$$

wherein $R^5$ is as defined with respect to formula (IV).

7. A process for the preparation of a compound as claimed in claim 1 which comprises reacting a compound of formula (VII):

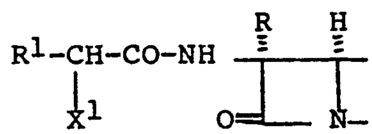

(VII)

wherein R and $R^1$ are as defined with respect to formula II:
with a compound of formula (IX):

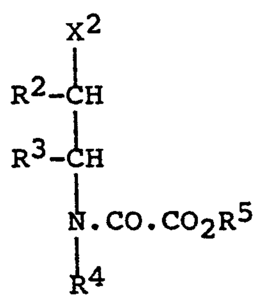

(IX)

wherein $R^2$, $R^3$, $R^4$, $R^5$ are as defined with respect to formula II,

and one of $X^1$ and $X^2$ represents amino, and the other represents an N-acylating derivative of a carbamic acid group, $-NH.CO_2H$.

8. A process for the preparation of a compound as claimed in claim 2 which comprises reacting a compound of formula (VIIa)

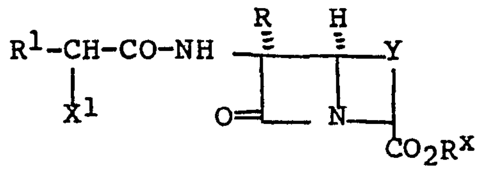

(VIIa)

wherein R, $R^1$, $R^x$ and Y are as defined with respect to formula (IV) with a compound of formula (IX)

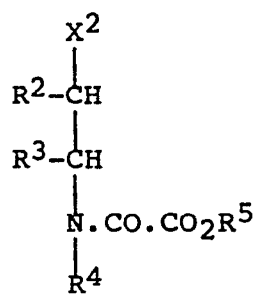

(IX)

wherein $R^2$, $R^3$, $R^4$ and $R^5$ are as defined with respect to formula (IV)
and one of $X^1$ and $X^2$ represents amino, and the other represents an N-acylating derivative of a carbamic acid group, $-NH.CO_2H$.

9. A process for the preparation of a compound as claimed in claim 1 which comprises reacting a compound of formula (XIII)

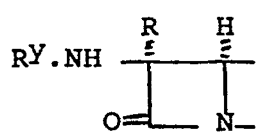

(XIII)

where R is as defined with respect to formula (II) and $R^y$ is hydrogen or a group which permits acylation to take place with a reactive N-acylating derivative of a compound of formula (XIV)

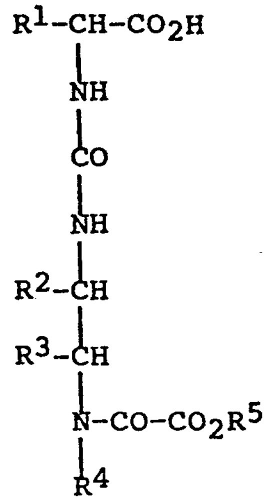

(XIV)

wherein $R^1$,$R^2$,$R^3$,$R^4$ and $R^5$ are as defined with respect to formula (II)

10. A process for the preparation of a compound as claimed in claim 2 which comprises reacting a compound of formula (XIIIa)

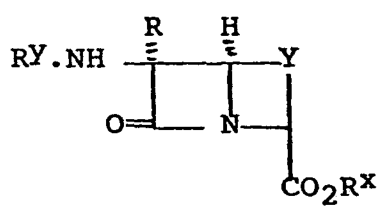

(XIIIa)

where R, $R^x$ and Y are as defined with respect to formula IV and $R^y$ is hydrogen or a group which permits acylation to take place with a reactive N-acylating derivative of a compound of formula (XIV)

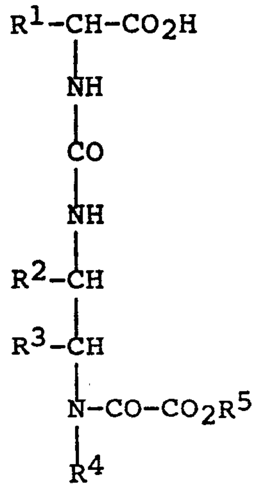

(XIV)

wherein $R^1$, $R^2$, $R^3$, $R^4$ and $R^5$ are as defined with respect to formula IV

11. A process as claimed in any one of claims 5,6,9 or 10 in which the N-acylating derivative is an acid halide, a symmetrical or mixed anhydride, an acid azide, an activated ester or the reactive intermediate formed by reaction _in situ_ with a condensing agent.

12. A compound as claimed, in claim 2 selected from the following or a salt thereof

6β-[D-2[[2-[_N_-ethyl-_N_-(4-nitrobenzyloxalyl)amino]-ethylamino]carbonylamino]-2-phenylacetamido]-6α-(methylthio)penicillanic acid or the benzyl ester thereof;

6β-[D-2-[[2-[N-ethyl-N-(4-nitrobenzyloxalyl)-amino]ethylamino]carbonylamino]-2-(3,4-diacetoxyphenyl) acetamido]-6α-formamidopenicillanic acid or the benzyl ester thereof;

6β-[D-2-[[2-[N-ethyl-N-(methyloxalyl)amino]-ethylamino]carbonylamino]-2-phenylacetamido]-6α-(methylthio)penicillanic acid or the benzyl ester thereof;

6β-[D-2-[[2-[N-ethyl-N-(4-nitrobenzyloxalyl)amino]-ethylamino]carbonylamino]-2-(3,4-diacetoxyphenyl)-acetamido]-6α-(methylthio)penicillanic acid or the benzyl ester thereof.

6β-[D-2-[[2-[N-ethyl-N-(methyloxalyl)amino]ethylamino]-carbonylamino]-2-(3,4-diacetoxyphenyl)acetamido]-6α-(methylthio)penicillanic acid or the benzyl ester thereof.

6β-[D-2-[[2-[N-ethyl-N-(methyloxalyl)amino]ethylamino]-carbonylamino]-2-(3,4-diacetoxyphenyl)acetamido]-6α-formamidopenicillanic acid or the benzyl ester thereof.

7β-[D-2-[[2-[N-ethyl-N-(4-nitrobenzyloxalyl)amino]-ethylamino]carbonylamino]-2-phenylacetamido]-7α-formamidocephalosporanic acid or the t-butyl ester thereof.

13. A process for the preparation of a β-lactam compound having the partial structure (I):

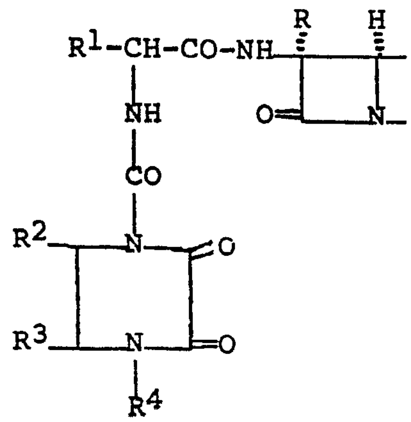

(I)

wherein: R represents $C_{1-6}$ alkylthio, arylthio, methoxy, hydroxymethyl, amino or formamido;

$R^1$ represents hydrocarbon or heterocyclyl;

$R^2$ and $R^3$ are the same or different and each represents hydrogen, optionally substituted $C_{1-6}$ alkyl, halogen, aryl,amino, hydroxy or $C_{1-6}$ alkoxy or $R^2$ and $R^3$ form the residue of a 5- or 6-membered carbocyclic or heterocyclic ring; and

$R^4$ represents hydrogen, or hydrocarbon;

which process comprises cyclising a compound of formula (II):

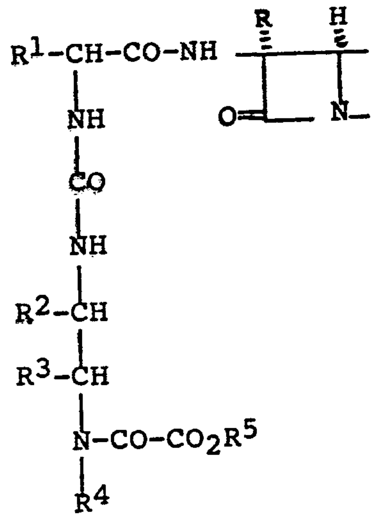

(II)

wherein R, $R^1$, $R^2$, $R^3$ and $R^4$ are as defined above and $R^5$ represents hydrogen or a group which allows the $-CO_2R^5$ group to effect N-acylation.

14. A process for the preparation of a compound of formula (III) or a salt thereof

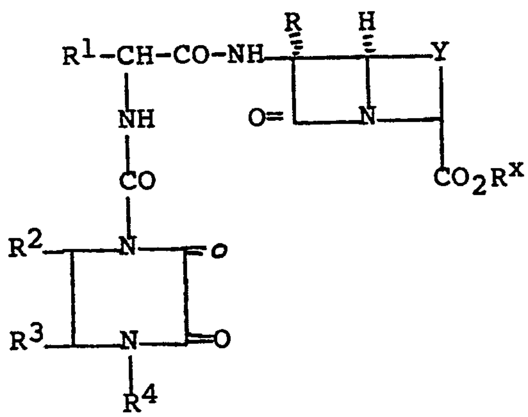

(III)

wherein R represents $C_{1-6}$ alkylthio, arylthio, methoxy, hydroxymethyl, amino or formamido;

$R_1$ represents hydrocarbon or heterocyclyl;

$R^2$ and $R^3$ are the same or different and each represents hydrogen, optionally substituted $C_{1-6}$ alkyl, halogen, aryl, amino, hydroxy or $C_{1-6}$ alkoxy or $R_2$ and $R_3$ form the residue of a 5- or 6-membered carbocyclic or heterocyclic ring; and

$R_4$ represents hydrogen, or hydrocarbon;

$R^x$ is hydrogen or a carboxyl blocking group;

and Y is:

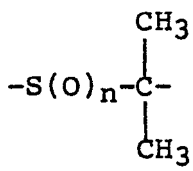 , $-S(O)_n-CH_2-$ ,

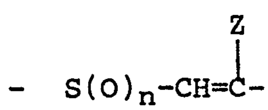 , 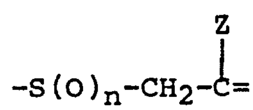 ,

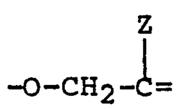 , 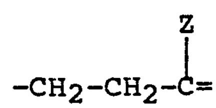 ,

wherein n is zero, 1 or 2 and Z represents hydrogen, halogen, or an organic group such as $C_{1-4}$ alkoxy, $-CH_2Q$ or $-CH=CH-Q$ wherein Q represents hydrogen, halogen, hydroxy, mercapto, cyano, carboxy, carboxylic ester, carbamoyloxy, $C_{1-4}$ alkyloxy, acyloxy, aryl, a heterocyclyl group bonded via carbon, a heterocyclylthio group or a nitrogen containing heterocyclic group bonded via nitrogen.

which process comprises cyclising a compound of formula (IV)

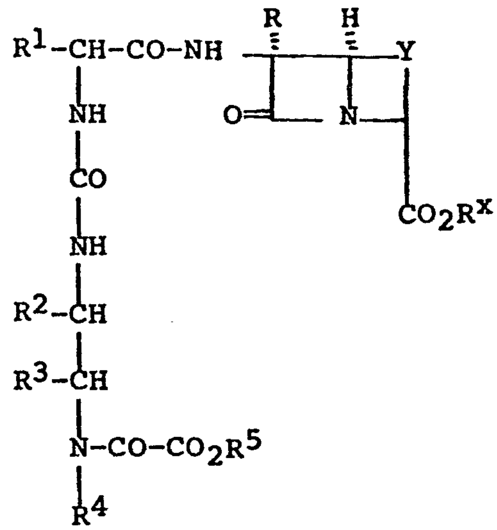

(IV)

wherein R, $R^1$, $R^2$,$R^3$,$R^4$,$R^x$ and Y are as defined above and $R^5$ represents hydrogen or a group which allows the $CO_2R^5$ group to effect acylation, and thereafter carrying out one or more of the following steps:

(i) converting a group $R^x$ into a different group $R^x$

(ii) converting a group Z into a different group Z.

15. A process as claimed in claim 14 in which Y is $-S-C(CH_3)_2-$ or $-S-CH_2-CZ=$ such that the compound of formula (III) is a derivative of penicillin or cephalosporin.

16. A process as claimed in claim 13 or claim 14 in which the cyclisation is carried out in a solvent in the presence of a base.

17. A process as claimed in claim 16 in which the solvent is water, $C_{1-6}$ alkanol, an ether, ester, chlorinated hydrocarbon, aromatic hydrocarbon or amide.

18. A process as claimed in claim 17 in which the solvent is water, methanol, ethanol, tehahydrofuran, dioxan, methyl acetate, ethyl acetate, dichloromethane chloroform, benzene, toluene or dimethyl formamide.

19. A process as claimed in claim 18 in which the base is an alkali metal hydroxide, carbonate, hydride or alkoxide, an amine, or a strong non-nucleophilic base.

20. A process as claimed in claim 19 in which the base is a tertiary amine.

21. A process as claimed in claim 20 in which the base is a trialkylamine, dialkylbenzylamine, 4-dimethylamino pyridine or methyl morpholine.

22. A process as claimed in claim 19 in which the non-nucleophilic base is 1,8-diazabicyclo[5.4.0]-undec-7-ene.

23. A process as claimed in any one of claims 13 to 22 in which R is formamido.

24. A process as claimed in any one of claims 13 to 23 in which $R^1$ is 3,4-dihydroxyphenyl, 3,4-diacetoxyphenyl or 3,4-dibenzyloxycarbonyloxyphenyl.

25. A compound of the formula (IX)

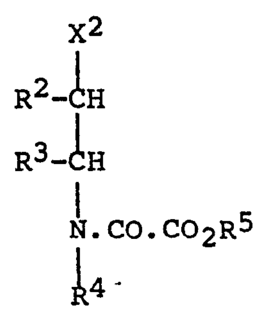

(IX)

wherein $R^2$, $R^3$, $R^4$ and $R^5$ are as defined with respect to formula (IV) and $X^2$ represents amino or an N-acylating derivative of a carbamic acid group, - $NH.CO_2H$.

26. A process for the preparation of a compound of formula (XIV):

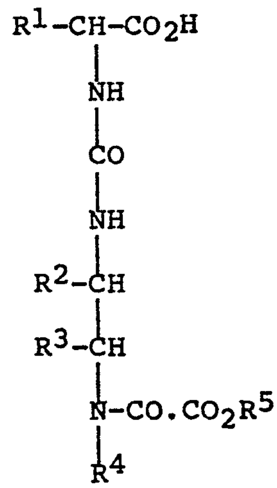

(XIV)

wherein $R^1$, $R^2$, $R^3$, $R^4$ and $R^5$ are as defined in claim 8 which comprises reacting a compound of formula (XV):

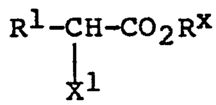

(XV)

with a compound of formula (IX):

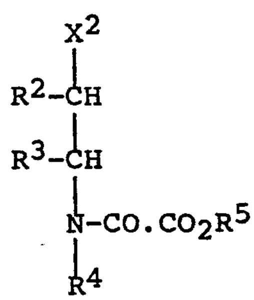

wherein $R^1$, $R^2$, $R^3$, $R^4$, $R^5$, $R^x$, $X^1$ and $X^2$ are as defined in claim 8; and if necessary removing any carboxyl blocking group.

27. A process for the preparation of a compound of formula (XIV):

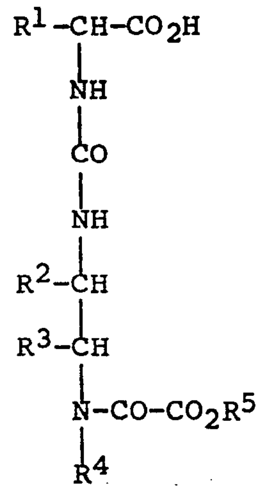

(XIV)

wherein $R^1$, $R^2$, $R^3$, $R^4$ and $R^5$ are as defined in claim 8 which comprises reacting a compound of formula (XVI):

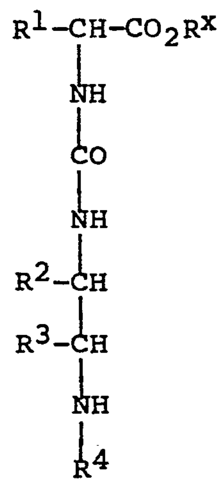

(XVI)

wherein $R^1$, $R^2$, $R^3$, $R^4$ and $R^x$ are as defined in claim 8 with a reactive N-acylating derivative of an acid formula (VI):

$R^5O_2C.CO_2H$ (VI)

wherein $R^5$ is as defined in claim 8; and thereafter if necessary removing any blocking group $R^x$.

28. A compound of the formula (XIV);

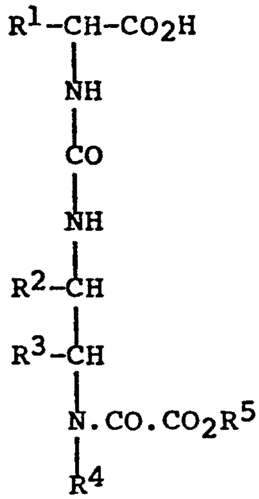

(XIV)

wherein $R^1$ is a 3,4-dihydroxyphenyl or protected 3,4-dihydroxyphenyl group and $R^2$, $R^3$, $R^4$ and $R^5$ are as defined in claim 8.

29. A compound as claimed in claim 28 in which $R^2$ and $R^3$ represent hydrogen and $R^4$ represents $C_{1-6}$ alkyl.

30. A compound as claimed in claim 28 selected from the following or a salt thereof:

D-2-[[2-[N-Ethyl-N-(4-nitrobenzyloxalyl)amino] ethylamino]carbonylamino]-2-(3,4-dihydroxyphenyl) acetic acid

D-2-[[2-[N-Ethyl-N-(4-nitrobenzyloxalyl)amino] ethylamino]carbonylamino]-2-(3,4-diacetoxyphenyl) acetic acid